(19) Europäisches Patentamt
European Patent Office
Office européen des brevets

(11) EP 1 447 102 B1

(12) **EUROPEAN PATENT SPECIFICATION**

(45) Date of publication and mention of the grant of the patent:
**04.06.2008 Bulletin 2008/23**

(51) Int Cl.:
*A61L 9/01* (2006.01) *A61L 9/14* (2006.01)

(21) Application number: **04100465.6**

(22) Date of filing: **09.02.2004**

(54) **Cyclohexylethan-1-yl ester mixtures as malodour counteractants and methods for using same**

Cyclohexylethan-1-yl-Ester-Zusammensetzungen zur Beseitigung unangenehmer Gerüche und zugehörige Verwendungsmethoden

Compositions de Cyclohexylethan-1-yl ester pour la suppression des mauvaises odeurs et procédés pour leur utilisation

(84) Designated Contracting States:
**DE ES FR GB IT**

(30) Priority: **11.02.2003 US 364234**

(43) Date of publication of application:
**18.08.2004 Bulletin 2004/34**

(73) Proprietor: **INTERNATIONAL FLAVORS & FRAGRANCES INC.**
**New York**
**New York 10019 (US)**

(72) Inventors:
- **Milan, Jolanda Bianca**
  **1082 EB Amsterdam (NL)**
- **O'Connor, Simon**
  **Cambridge CB1 3EQ (GB)**
- **Jacob, Tim J.C.**
  **Cardiff CF24 3RE (GB)**
- **Williams, Virgil A.G.**
  **Leonardo,**
  **Monmouth County,**
  **NJ 07737 (US)**
- **Nicoll, Stephen P.**
  **Ramsey,**
  **Bergen County,**
  **NJ 07446 (US)**
- **Boden, Richard M.**
  **Ocean,**
  **Monmouth County,**
  **NJ 07712 (US)**
- **Wang, Liwei**
  **Cardiff CF23 6DT (GB)**

(74) Representative: **Goodfellow, Hugh Robin**
**Carpmaels & Ransford**
**43-45 Bloomsbury Square**
**London WC1A 2RA (GB)**

(56) References cited:
**EP-A- 1 167 507**
**WO-A-00/27442**
**GB-A- 1 545 561**
**US-A- 4 719 105**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 447 102 B1

## Description

Our invention is directed to a mixture consisting essentially of 1-cyclohexylethan-1-yl butyrate having the structure:

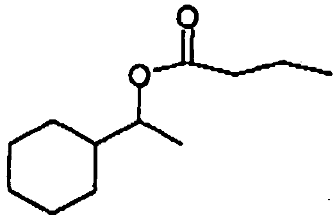

and 1-cyclohexylethan-1-yl acetate having the structure:

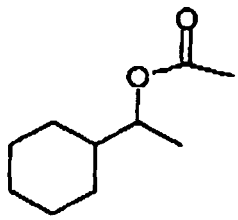

the weight ratio of 1-cyclohexylethan-1-yl butyrate:1-cyclohexylethan-1-yl acetate being from 20:80 up to 50:50, in the substantial absence of the compounds: 1-cydohexytcthan-1-ol having the structure:

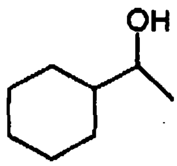

1-(4'-methylethyl)cyclohexylethan-1-yl propionate having the structure:

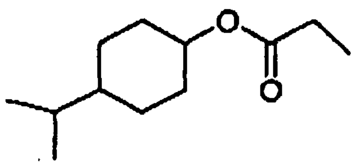

and 2'-hydroxy-1'-ethyl(2-phenoxy)acetate having the structure:

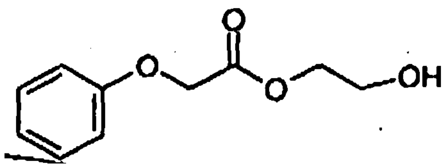

The mixture may optionally have a substantial absence of any additional fragrance substances or malodour counteractant substances. The ester mixture is synergistically effective for its ability to counteract a malodour (1) emanating from a malodourous solid or liquid source into a 3-space proximate the solid or liquid source or (2) present in a malodourous

air 3-space. The malodourous solid or liquid source and the malodourous air 3-space may or may not contain an additional desirable fragrance. The ester mixture is used as a malodour counteractant by introducing into the 3-space containing the malodour, or proximate the solid or liquid malodourous source an effective malodour-counteracting quantity and concentration of the ester mixture as a single dose, as a continuous dose over a malodour-counteracting period of time, or as periodic doses over a malodour-counteracting period of time whereby the perceived total malodour intensity is substantially reduced or eliminated. When the 3-space contains, in addition to the malodour, a desirable fragrance, the addition of an effective malodour-counteracting quantity and concentration of the synergistically-effective ester mixture as a single dose, as a continuous dose over a malodour-counteracting period of time, or as periodic doses over a malodour-counteracting period of time to the 3-space does not eliminate or substantially reduce the perceived odour intensity of the desired fragrance. The term "3-space" is herein intended to mean "3-dimensional volume, as measured using "x", "y" and "z" coordinates".

## BACKGROUND

A wide variety of solid, liquid and gaseous functional materials including body deodorants, anti-perspirants, anti-perspirant/body deodorant devices, air fresheners which include air freshening devices, and solid and liquid air freshening and room freshening compositions, room deodorants, herbicides, antiviral compositions, fungicides, bactericides, parasiticides, insecticides, depilatory compositions, bleach compositions, hard surface-cleaning compositions, skin cleansing compositions, antimicrobial nail preparations including anti-fungal nail lacquers, hair setting compositions, hair conditioning compositions, trichological lotions, detergent compositions, soap compositions, sunscreen compositions, fabric stain-removal compositions, fabric conditioning compositions, fabric anti-wrinkle compositions, skin lightening compositions, steam iron aroma compositions including stress relief compositions, candle compositions, plant growth regulating compositions, plant growth stimulating compositions, fertilizer compositions, insect attractant compositions, insect repelling compositions, drain cleaning compositions and molluskicide compositions have been developed that, although useful for their respective purposes, on use thereof emanate odours which are offensive to the human sense of smell. In addition, a number of defined 3-spaces the use of which is required for various business and service operations and personal matters including indoor gymnasiums, indoor sporting event arenas, locker rooms, hair salons, nail salons, tanning salons, beauty salons, tattoo parlors, pig pens, chicken coops, cow barn enclosures, horse barn enclosures, indoor fresh fish markets, plant processing factory rooms, clothing dry cleaning rooms, garment laundry interiors, rooms containing in-use animal litter containers, abattoirs, cattle cars, zoo animal pens, morgues, autopsy rooms, lavatories, medical patient care rooms, hospital wards and dental patient care rooms have continuously prevailing odours which are offensive to the human sense of smell. Such odors which are offensive to the human sense of smell are caused by aliphatic halohydrins, aliphatic amines, aliphatic N-oxides, dialkylamines, cycloaliphatic amines, cycloaliphatic N-oxides, cyclo-olefinic amines, cyclo-olefinic N-oxides, cycloaromatic amines, cycloaromatic N-oxides, hydroxyalkylamines, imine compounds, amide compounds, amino acids, polypeptides, modified antimicrobial proteins, diureides, nitriles, aliphatic mercaptans, cycloaliphatic mercaptans, mercaptoalkanoic acids, mercaptoalkanoic acid esters, aliphatic monosulfides, disulfides, trisulfides, sulfur oxides, sulfones and sultones, cycloaliphatic monosulfides, disulfides, trisulfides, sulfur oxides, sulfones and sultones, cyclo-olefinic monosulfides, disulfides, trisulfides, sulfur oxides, sulfones and sultones, cycloaromatic monosulfides, disulfides, trisulfides, sulfur oxides, sulfones and sultones, alkali metal sulfites, bisulfites and metabisulfites, isothiocyanates, thiocyanates, dithiocyanates, isothiazolones, isothiazolinones, thiodiazinethiones, halosulfamates, aryl sulfonamides, lower aliphatic carboxylic acids, phenols, phosphines, aliphatic phosphites and phosphonates, cycloaliphatic phosphites and phosphonates, arsines, lower alcohols, lower ketones, hops, hops acids, aryl pyrazoles, oxazolines, isocyanurates, biguanides, extracts of krameria, hydantoins, pyrollidones, pyrollidone carboxylic acids, pyrollidone carboxylic acid esters, nitrophenols, N-substituted aspartic acids and pyrethroids. Compounds of these classes that have unpleasant odours are referred to herein as malodour compounds.

The aforementioned functional materials are known to include and/or have applied thereto or in a 3-space proximate thereto, fragrance materials that are intended to provide pleasant fragrances which mask the malodour. In addition, the aforementioned defined 3-spaces are known to have introduced therein fragrance materials that are intended to provide pleasant fragrances which mask the malodour present therein and/or introduced thereto. The masking effect is provided by one of two mechanisms. In the first mechanism, the masking fragrance blends with the malodour compound or compounds in an effort to provide a different and more desirable aroma. In the second mechanism the masking fragrance is employed in a large quantity to overwhelm the compound or composition responsible for the malodour.

Both types of mechanisms have serious disadvantages. Neither perfume completely eliminates the perception of malodour and, accordingly, there is a tendency to use increasing quantities and concentrations of fragrance in an effort to eliminate the perception of malodour. Furthermore, the masking effect is an additive effect and so the total odor level in the malodour-masked functional product or in the malodour-masked defined 3-space is increased by consumption of the perfume. Even though the perfume so used may be very pleasant at low concentration, the total odour level in the 3-space proximate the in-use functional product and the total odour level in the aforementioned defined 3-space at

the relatively high concentrations required to achieve even moderate masking of the malodour will itself be offensive to the human sense of smell.

Attempts have been made to overcome the aforementioned disadvantages using cyclohexyl-1-ethyl ester substances.

Schleppnik, U.S.Patent No. 4,622,221 discloses a method of counteracting a malodour in air caused by a compound selected from the group consisting of lower carboxylic acids, thiols, thiophenols, phenols, lower amines, phosphines and arsines. The method of U.S.Patent No. 4,622,221 comprises introducing into the air an effective malodour-counteracting amount of cyclohexyl-1-ethyl-n-butyrate or cyclohexyl-1-ethyl acetate whereby the perceived total odor intensity in the air is reduced and the perceived malodour intensity in the air is substantially eliminated. The Schleppnik patent does not, however, disclose or suggest the synergistically-effective cyclohexylethan-1-yl ester mixtures of our invention and their unexpected, unobvious and advantageous properties and use as malodour counteractants as measured physiologically and psychometrically.

O'Connor, U.S. Patent No. 6,432,891 discloses a method of counteracting a malodour in a solid or liquid soap or detergent caused by a compound selected from the group consisting of lower carboxylic acids, thiols, thiophenols, lower amines, phosphines, arsines, lower alcohols, and lower ketones. The method disclosed by U.S. Patent 6,432,891 comprises introducing into the solid or liquid soap or detergent an effective malodour-counteracting amount of a malodour-counteracting compound selected from the group consisting of 1-cyclohexyl-ethyl-butyrate, 1-cyclohexyl-ethyl-acetate, 1-cyclohexyl-ethanol, 4-isopropyl-cyclohexyl-propionate, and phenoxyacetic acid 2-hydroxy-ethyl ester. U.S. Patent 6,432,891 further discloses that the perceived total odour intensity in the solid or liquid soap or detergent is reduced, and the perceived malodour intensity in the solid or liquid soap or detergent is substantially eliminated. U.S. Patent 6,432,891 includes examples of the use of mixtures of (i) 1-cyclohexyl-ethyl-acetate and 4-isopropyl-cyclohexyl-propionate in weight rations of 20:80, 50:50 and 80:20 and (ii) 1-cyclohexyl-ethyl-acetate, 1-cyclohexyl-ethanol and 4-isopropyl-cyclohexyl-propionate in attempts to counteract malodours in perfumed shower creams.

The O'Connor patent does not, however, disclose or suggest the synergistically-effective cyclohexylethan-1-yl ester mixtures of our invention and their unexpected, unobvious and advantageous properties and use as malodour counteractants as measured physiologically and psychometrically.

In addition, prior sales have been effected for use as perfumed malodour counteracting compositions of mixtures of 1-cyclohexyl-ethyl-butyrate, 1-cyclohexyl-ethyl-acetate, 1-cyclohexyl-ethanol and 4-isopropyl-cyclohexyl-propionate. Such prior-sold compositions, however, are different in kind from the synergistically-effective cyclohexylethan-1-yl ester mixtures of our invention which have unexpected, unobvious and advantageous properties and use as malodour counteractants as measured physiologically and psychometrically.

## THE INVENTION

Our invention is directed to a mixture consisting essentially of 1-cyclohexylethan-1-yl butyrate having the structure:

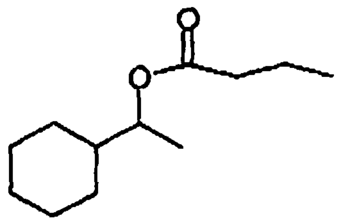

and 1-cyclohexylethan-1-yl acetate having the structure:

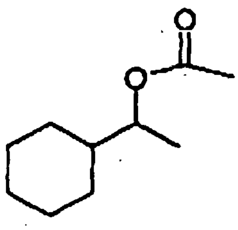

the weight ratio of 1-cyelohexylethan-1-yl butyrate:1-cyclohexylethan-1-yl acetate being from 20:80 up to 50:50, in the substantial absence of the compounds:
1-cyclohexylethan-1-ol having the structure:

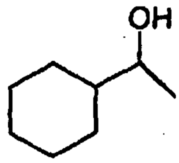

1-(4'-methylethyl)cyclohexylethan-1-yl propionate having the structure:

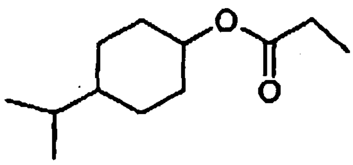

and 2'-hydroxy-1'-ethyl(2-phenoxy)acetate having the structure:

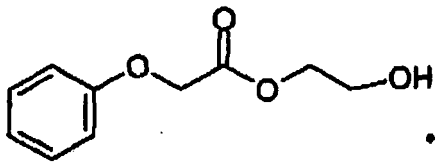

The mixture may
optionally have a substantial absence of any additional fragrance substances or other malodour counteractant substances, The term, "substantial absence" of a substance is herein intended herein to mean "less than about 1% by weight" of the substance.

Our invention is also directed to processes for counteracting malodours, as set out in the appended claims.

In one embodiment, when the 3-space contains, in addition to the malodour, a desirable fragrance, the addition of an effective malodour-counteracting quantity and concentration of the synergistically-effective ester mixture of our invention as a single dose, as a continuous dose over a malodour-counteracting period of time, or as periodic doses over a malodour-counteracting period of time to the 3-space does not eliminate or substantially reduce the perceived odour intensity of the desired fragrance.

The processes of our invention may thus effect malodour coverage of a wide variety of solid, liquid and gaseous functional materials including body deodorants, anti-perspirants, anti-perspirant-deodorant devices, air fresheners including air freshening devices and solid and liquid air freshening compositions further including room freshener compositions, room deodorants, herbicides, antiviral compositions, fungicides, bactericides, parasiticides, insecticides, depilatory compositions, bleach compositions, hard surface-cleaning compositions, skin cleansing compositions, antimicrobial nail preparations including anti-fungal nail lacquers, hair setting compositions, hair conditioning compositions, trichological lotions, skin lightening compositions, detergent compositions, soap compositions, sunscreen compositions, fabric stain removal compositions, fabric conditioning compositions, fabric anti-wrinkle compositions, steam ironing aroma compositions including stress relief compositions, candle compositions, plant growth regulating compositions, plant growth stimulating compositions, fertilizer compositions, insect attractant compositions, insect repelling compositions, drain cleaning compositions and molluskicide compositions. Such functional materials although useful for their respective purposes, on use thereof emanate odours which are offensive to the human sense of smell.

In addition, the processes of our invention may effect malodour coverage in various defined air 3-spaces which have continuously prevailing malodours of constant or variable perception which are offensive to the human sense of smell, for example, indoor gymnasiums, indoor sporting event arenas such as basketball arenas, locker rooms, abattoirs, indoor sporting courts such as handball courts, hair salons, nail salons, tanning salons, beauty salons, tattoo parlors,

pig pens, chicken coops, cow barn enclosures, indoor fresh fish markets, fishing boat interiors, horse barn enclosures, rooms containing in-use animal litter containers, kitchens, restaurants, processed food preparation spaces, clothing dry cleaning rooms, garment laundry interiors, cattle cars, plant processing factory rooms, domestic pet shops, zoo animal pens, morgues, autopsy rooms, lavatories, medical patient care rooms, hospital wards and dental patient care rooms.

The aforementioned malodours are caused by chemical compounds of the following classes: aliphatic halohydrins, aliphatic amines, aliphatic N-oxides, dialkylamines, cycloaliphatic amines, cycloaliphatic N-oxides, cyclo-olefinic amines, cyclo-olefiaic N-oxides, cycloaromatic amines, cycloaromatic N-oxides, hydroxyalkylamines, imine compounds, amide compounds, amino acids, polypeptides, modified antimicrobial proteins, diureides, nitriles, aliphatic mercaptans, cycloaliphatic mercaptans, mercaptoalkanoic acids, mercaptoalkanoic acid esters, aliphatic monosulfides, disulfides, trisulfides, sulfur oxides, sulfones and sultones, cycloaliphatic monosulfides, disulfides, trisulfides, sulfur oxides, sulfones and sultones, cyclo-olefinic monosulfides, disulfides, trisulfides, sulfur oxides, sulfones and sultones, cycloaromatic monosulfides, disulfides, trisulfides, sulfur oxides, sulfones and sultones, alkali metal bisulfites, sulfites and metabisulfites, isothiocyanates, thiocyanates, dithiocyanates, isothiazolones, isothiazolinones, thiodiazinethiones, halosulfamates, aryl sulfonamides, lower aliphatic carboxylic acids, phenols, phosphines, aliphatic phosphites and phosphonates, cycloaliphatic phosphites and phosphonates, arsines, lower alcohols, lower ketones, hops, hops acids, aryl pyrazoles, oxazolines, isocyanurates, biguanides, extracts of krameria, hydantoins, pyrollidones, pyrollidone carboxylic acids, pyrollidone carboxylic acid esters, nitrophenols, N-substituted aspartic acids and pyrethroids.

The synergistically-effective ester compositions of our invention substantially eliminate the perception of such malodours, while simultaneously refraining from reduction of the perception of pleasant fragrance aromas emanating from the same source or from the proximity of said source.

Examples of functional products which are compositions, effective specific malodourous ingredients or classes of ingredients contained in and/or emanated from said functional products and exemplary defined air 3-spaces where such functional products are used, together with U.S. Patent references setting forth specific examples of the utilities of such functional products are set forth in the following Table I:

**TABLE I**

| Functional Product | U. S. Patent Reference | Ingredient or Ingredient Class | Defined Air 3-Space |
|---|---|---|---|
| clothing stain removal composition | 6,495,510 | diethyl-n-dodecyl amine oxide | clothing dry cleaning rooms |
| bleach composition | 6,498,133 | acetonitrile | clothing garment laundry interior |
| Skin lightening composition | 6,497,860 | sodium bisulfite | beauty salon |
| herbicide | 6,495,492 | 4-chloro-3-(4-cyano-2-fluoro-5-phenoxyphenyl)-1-methyl-5-trifluoromethyl-1H-pyrazole | plant greenhouse |
| viruside | 6,468,521 | silver complex of trihydroxymethylaminomethane | hospital ward |
| fungicide | 6,495,575 | synergistically effective amount of valinamide and fluazinam | plant greenhouse |
| bactericide | 6,207,274; 6,475,976 | 4-chloro-5-methyl-2,3-dithiolane; polyhexamethylene-4-biguanide | kitchen |
| parasiticide | 6,265,350 | 1-methylthio(ethylideneamino)-N-methyl-N-(morpholinothio)carbamate | pet shop |
| insecticide | 4,171,340 | 0,0-dimethyl S-(1,2-dicarboethoxyethyl)-dithiophosphate | processed food preparation room |
| depilitory preparation | 6,479,043 | calcium thioglycolate | hair salon |
| hard surface cleaning preparation | 6,440,925 | dodecyldimethylamine oxide | kitchen |
| skin cleansing preparation | 6,491,933 | sodium cocoyl isethionate | beauty salon |

(continued)

| Functional Product | U. S. Patent Reference | Ingredient or Ingredient Class | Defined Air 3-Space |
|---|---|---|---|
| anti-fungal nail lacquer composition | 6,495,124 | macrocyclic keto-lactones | nail salon |
| body deodorant compositon | 6,325,565 | L-lysine hexadecylamide | locker room |
| anti-perspirant composition | 6,325,565 | zirconyl hydroxychloride-glycine complex | locker room |
| steam ironing aroma composition | 6,495,172 | witch hazel | laundry room interior |
| antimicrobial nail preparation | 6,479,039; 6,495,124... | N,N-diethylammonium chloride plus phenol or biguanide; econazole or 1-[2-[(4-chlorophenyl)-methoxy]-2-(2,4-dichlorophenyl)ethyl]-1*H*-imidazole | nail salon |
| hair setting composition | 6,479,042 | thioglycolic acid | hair salon |
| hair conditioning composition | 6,491,902 | polyvinyl amines | hair salon |
| trichological lotion | 6,479,059 | cysteine | hair salon |
| detergent<br>soap | 6,491,728<br>6,479,456 | $C_{14}$-alkylethoxysulfonic acid<br>3,4,4'-trichlorocarbanilide | lavatory<br>lavatory |
| sunscreen composition | 6,485,713 | p-hydroxydiphenylsulfonate | tanning salon |
| fabric conditioning compositions | 6,482,787; 6,491,840 | cyclic amine-based polymers; N,N"-dioleoyldiethylenetriamine and 2,4,6-trimethyl pyridine | clothing dry cleaning rooms |
| candle compositions | 6,111,055; 5,879,694 | ester-terminated dimer acid-based Polyamide; oxidized hydrocarbon oil | restaurant |
| plant growth-regulating composition | 6,444,614 | N-(1,2-dicarboxyethyl)aspartic acid | plant greenhouse |
| plant growth stimulating composition | 6,444,614 | N,N'-1,2-ethanediylbis-aspartic acid | plant greenhouse |
| fertilizer composition | 6,500,222 | diureides | plant greenhouse |
| insect attractant | 5,928,634; 6,410,567 6,410,567 | sucrose, fructose, maltose and the lithium salt of saccharin; disparlure plus a tetrapyrrole | plant greenhouse |
| insect repellent | 6,451,844; 6,306,415 6,306,415 | menthyl-2-pyrollidone-5-carboxylate; 1:1:1 octanoic acid, nonanoic acid and decanoic acid | kitchen |
| drain cleaning composition | 6,479,444 | $C_{14-18}$alkyl sulfobetaine plus NaOCl | kitchen |
| room freshener composition | 4,622,221 | d-limonene | kitchen |

(continued)

| Functional Product | U. S. Patent Reference | Ingredient or Ingredient Class | Defined Air 3-Space |
|---|---|---|---|
| malodourous plant processing environment | 6,491,962 | amino acids plus sodium hypochlorite | plant processing factory room |
| molluskicide | 6,265,350 | 1,3-oxazolines and 1,3-thiazolines | fishing boat interior |

The specification of each of the patents a listed in Table I is herein incorporated by reference as if set forth in their entirety.

When used in conjunction with malodourous solid or liquid functional products the synergistically effective ester malodour counteracting mixtures of our invention may comprise from about 0.01% by weight up to about 15.0% by weight of the solid or liquid functional product as set forth and as specifically exemplified in Example I, preferably from about 0.025% up to about 10% and more preferably from about 0.05% up to about 4.0%.

When used in conjunction with malodourous gaseous functional products, the synergistically effective ester malodour-counteracting mixtures of our invention may comprise from about 0.01 up to about 1 mg per cubic meter of the air 3-space which contains the malodour to be counteracted.

Specific details concerning the functional products, soaps and detergents, are set forth in U.S. Patent 6,432,891. Specific details concerning the air freshener composition functional product are set forth in U.S. Patent 4,622,221.

Examples of functional products which are air freshener devices used in the practice of our invention together with U.S. Patent references setting forth specific examples of the utilities of such air freshener devices are set forth in the following Table II:

**Table II**

| Air Freshening Device | U.S. Patent Reference |
|---|---|
| Night Light Air Freshener | 6,478,440 |
| Light Bulb | 4,184,099 |
| Room Freshener | 4,837,421 |

The specification of each of the patents listed in Table II is herein incorporated by reference.

An example of a body deodorant/anti-perspirant device used in the practice of our invention is that shown in Figure 7 of U.S. Patent 6,325,565 and described in detail in said U.S. Patent 6,325,565.

The synergistically effective ester composition of our invention may be delivered to the location of its use by means known to those having ordinary skill in the art, including (i) directing to the desired site the ester composition in the form of a gas phase vapor, (ii) spraying the ester composition of our invention as an aerosol continuously, or as pulsed doses, or as an initial dose, (iii) forming microcapsules containing the ester composition surrounded by a wall of, for example, gelatin formed by a coacervation process and then delivering the microcapsules to the site of use where the ester compositions of our invention are controllably released or (iv) in the case of a solid malodorous porous functional product, injecting into the interstices of the solid functional product as an initial dose, or in continuous doses or in pulsed doses, the ester composition of our invention. When the delivery of the synergistically-effective ester composition to the desired location is carried out by directing a gas phase vapor to the desired site, the apparatus and process disclosed in Young et al., Application for U.S. Letters Patent Serial No. 10/212,349 filed on August 5, 2002 may be used. When the delivery of the synergistically-effective ester composition of our invention is carried out by means of introduction of control-release microcapsules to the desired location, microcapsules produced according to the disclosures of U.S. Patent 6,491,902 or U.S. Patent 6,485,736 may be used.

When the delivery of the synergistically-effective ester composition of our invention is carried out in pulses, the time period for each pulse is herein indicated as "pulse duration" and the time between the pulses is herein indicated as "inter-stimulus intervals" or 'ISI". The process of our invention wherein the synergistically-effective ester composition is delivered to the desired site in pulses or "periodic doses" over a malodour-counteracting period of time is governed by the model:

$$Z = Z_o + A(1-B^X) + C(1-D^Y)$$

wherein **Z** is the percentage of odour pulses correctly identified;
**Y** is the pulse duration measured in units of time;
**X** is the inter-stimulus interval measured in units of time;
$\mathbf{Z_o}$ is a measure, as a scaled value of from -100 up to 100 of the sensitivity of the subjects to the odour detected;
**A** and **B** are parameters, each defining the effects of the inter-stimulus interval, **X**, and stimulus frequency, $\phi$, on the percentage of odour pulses correctly identified, **Z,** and each is a measure of the degree of adaptation to malodours and habituation as measured by the tangent slope to the **X-Z** curve at a given point, $(X_1, Z_1)$, $\nabla F(X_1, Z_1)$;
**C** is a concentration parameter and is a measure of the magnitude of the tangent slope to the **Y-Z** curve at a given point, $(Y_1, Z_1)$, $\nabla F(Y_1, Z_1)$; **C** and **D** are parameters, each being measures of the effects of pulse duration, **Y**, and the inter-stimulus interval, **X** on the percentage of odour pulses correctly identified, **Z,** and each is a measure of the angle of inclination, $\theta$, of the normal to the tangent plane to the **X-Y-Z** surface at a point $(X_1,Y_1,Z_1)$ wherein $\theta = \arccos[|\nabla F (X_1, Y_1,Z_1).|k|/\{||\nabla F (X_1,Y_1,Z_1)||\}]$ with |**k** representing the "z" axis vector and (i) in the case of the weight ratio range of the 1-cyclohexylethan-1-yl butyrate:1-cyclohexylethan-1-yl acetate being from about 20:80 up to about 80:20, $\mathbf{Z_o}$ being in the range of from about -45 up to about -15; **A** being in the range of from about 50 up to about 65; **B** being in the range of from about 0.93 up to about 0.97; **C** being in the range of from about 68 up to about 71; **D** being in the range of from about 0.98 up to about 0.99; and with (ii) in the case of the weight ratio range of the 1-cyclohexylethan-1-yl butyrate:1-cyclohexylethan-1-yl acetate being in the range of from about 50:50 up to about 80:20, $\mathbf{Z_o}$ being in the range of from about -45 up to about -25; **A** being in the range of from about 55 up to about 65; **B** being in the range of from about 0.95 up to about 0.97; **C** being in the range of from about 68 up to about 70; and **D** being in the range of from about 0.98 up to about 0.99.

A detailed discussion of the model:

$$= Z_o + A(1-B^X) + C(1-D^Y)$$

as it applies to individual malodour molecules and individual fragrance molecules is set forth in Jacob et al., "Psychometric Evaluation of Responses to Pleasant and Malodour Stimulation in Human Subjects; Adaptation, Dose Response and Gender Differences", Int. J. Psychophysiology, (2003). An additional discussion of the model as it applies to olfaction is set forth in Wang et al., "'The correlation between physiological and psychological responses to odor stimulation in human subjects" Clinical Neurophysiology 113 (2002) 542-551 at page 546.

## BRIEF DESCRIPTION OF THE DRAWINGS

**Figure 1** is a schematic diagram of the olfactometer used in carrying out the procedures of Example I.

**Figure 2** is a schematic diagram of the left side of the head of a human mammal indicating placement of electrodes and related brain regions in accordance with "The International 10-20 System of Electrode Placement" as utilized for the procedures and as described in detail in Example I.

**Figure 3** is a schematic diagram of the top view of the head of the human mammal of Figure 2.

**Figure 4A** is a set of bar graphs showing the measurements of the counteraction of a valeric acid malodour by a 20:80 mixture of cyclohexylethan-1-yl butyrate and 1-cyclohexylethan-1-yl acetate as a function of the magnitude of the standardized organoleptic event-related potential, "OERP" in Example I.

**Figure 4B** is a set of bar graphs showing the measurements of the counteraction of a valeric acid malodour by a 20:80 mixture of cyclohexylethan-1-yl butyrate and 1-cyclohexylethan-1-yl acetate as a function of % psychometric-cognitive recognition of odor or "% perception" in Example I.

**Figure 5A** is a set of bar graphs showing the measurements of the counteraction of a valeric acid malodour by a 50:50 mixture of cyclohexylethan-1-yl butyrate and 1-cyclohexylethan-1-yl acetate as a function of the magnitude of the standardized organoleptic event-related potential, "OERP" in Example I.

**Figure 5B** is a set of bar graphs showing the measurements of the counteraction of a valeric acid malodour by a 50:50 mixture of cyclohexylethan-1-yl butyrate and 1-cyclohexylethan-1-yl acetate as a function of % psychometric-cognitive recognition of odor or "% perception" in Example I.

**Figure 6A** is a set of bar graphs showing the measurements of the counteraction of a valeric acid malodour by a 80:20 mixture of cyclohexylethan-1-yl butyrate and 1-cyclohexylethan-1-yl acetate as a function of the magnitude of the standardized organoleptic event-related potential, "OERP" in Example I.

**Figure 6B** is a set of bar graphs showing the measurements of the counteraction of a valeric acid malodour by a 80:20 mixture of cyclohexylethan-1-yl butyrate and 1-cyclohexylethan-1-yl acetate as a function of % psychometric-cognitive recognition of odor or "% perception" in Example I.

**Figure 7A** is a set of bar graphs showing the measurements of the counteraction of an amyl acetate "pleasant aroma" by a 20:80 mixture of cyclohexylethan-1-yl butyrate and 1-cyclohexylethan-1-yl acetate as a function of the magnitude of the standardized organoleptic event-related potential, "OERP" in Example I.

**Figure 7B** is a set of bar graphs showing the measurements of the counteraction of an amyl acetate "pleasant aroma" by a 20:80 mixture of cyclohexylethan-1-yl butyrate and 1-cyclohexylethan-1-yl acetate as a function of % psychometric-cognitive recognition of odor or "% perception" in Example I.

**Figure 8A** is a set of bar graphs showing the comparison of the % inhibition of OERP for ester mixtures of our invention vs. the individual components of the ester mixtures of our invention as set forth in detail in Example I.

**Figure 8B** is a set for bar graphs showing the comparison of the % inhibition of perception for ester mixtures of our invention vs. the individual components of the ester mixtures of our invention as set forth in detail in Example I.

**Figure 9A** is a dose-stimulus-frequency set of bar graphs for the malodour, n-butyric acid shown in three dimensions.

**Figure 9B** is a dose-stimulus-frequency set of bar graphs for the "pleasant" odour, amyl acetate, shown in three dimensions.

**Figure 9C** is a set of adaptation curves for the malodour, n-butyric acid with the vertical axis being the "Z" axis for % odor pulses correctly defined and the horizontal axis being the "X" axis for inter-stimulus interval.

**Figure 9D** is a set of adaptation curves for the "pleasant" odour, amyl acetate, with the vertical axis being the "Z' axis for % odour pulses correctly defined and the horizontal axis being the "X" axis for inter-stimulus interval.

## EXAMPLE I AND DETAILED DESCRIPTION OF FIGURES 1-8B OF THE DRAWINGS

This example is indicative of properties of different proportions of binary combinations of cyclohexylethan-1-yl butyrate and cyclohexylethan-1-yl acetate against the malodour valeric acid.

Mixtures of cyclohexylethan-1-yl butyrate and cyclohexylethan-1-yl acetate, hereinafter referred to as the "ester mixtures" were used as the counteractant and n-valeric acid, hereinafter referred to as "VA" as the malodour. The mixtures were diluted with dipropylene glycol to final concentrations of 0.01% for each ester mixture and 2% for the VA.

Three weight ratio combinations of ester mixture components were tested: cyclohexylethan-1-yl butyrate:cyclohexylethan-1-yl acetate at 20:80, 50:50 and 80:20. For each mixture between 7-12 subjects were used, attempting to balance the genders, giving a total of 152 subjects. The single individual ester components, cyclohexylethan-1-yl butyrate and cyclohexylethan-1-yl acetate were repeated at the concentration, 0.01% for the purposes of comparison.

The ability of the 50:50 mixture of the cyclohexylethan-1-yl butyrate and cyclohexylethan-1-yl acetate was tested against a pleasant odour, amyl acetate, as a control.

## Delivery of odour

As shown in **Fig. 1,** odours are delivered to the nostril **103** by using an olfactometer system **10.** There are four teflon-lined solenoid three-way valves, **12A**, **12B, 15A** and **15B** (Cole Palmer, Bishops Stortford, UK) in the system. The solenoid valves are switched by Darlington drivers under computer control via the digital output of an A/D converter (CED 1401, Cambridge, UK).

Through lines **11A, 11B** and **11C,** air is pumped by a microprocessor-controlled pump (J. D. Technical Services, Old Ynysybwl, Wales, UK). The airflow is split into three directions and connected in parallel to three flow meters (Platon Flowbits, Basingstoke, UK), set to 1 L/min each. The outputs of the flow meters are diverted to three reservoir destinations,

respectively, (i) **13A** or **14A,** via lines **16A** or **18A,** respectively, (ii) **13B** or **14B** via lines **16B** or **18B,** respectively, and (iii) **14C** via line **160C.** Line **160C** is connected to a glass reservoir **14C** containing warm water, and are then joined with the output of other two sets of reservoirs **13A-13B** and **14A-14B.** The other two outlets of the flow meters are connected via lines **11A** and **11B** to valves **12A** and **12B,** respectively. Air flowing through each of the two sets of two parallel lines **16A-18A,** and **16B-18B,** leaving each of valves **12A** and **12B,** respectively, is fed to two glass reservoirs containing (i) either warm water in the case of reservoir **14A** or VA or amyl acetate in the case of reservoir **13A** or (ii) warm water in the case of reservoir **14B** or one of the ester mixtures in the case of reservoir **13B,** and then passed via lines **17A** and **19A** to valve **15A,** or via lines **17B** and **19B** to valve **15B.** The airflow leaving valves **15A** and **15B** is then combined with the airflow emanating from reservoir **14C** passing through line **100C** from the output of airflow through line **11C** and the combined airflow is passed through line **102** into one of the nostrils **103.** The nostril **103** is blocked by a self-expanding foam bung with the odour delivery tube passing through its centre. This prevents back-flow and forces the air to pass through the nasopharynx into the oral cavity.

Odor concentration is altered by varying the pulse duration; that is the length of time that the air flow is diverted through odor reservoir **13A** and/or **13B** directed by means of appropriate adjustment of the solenoid valves **12A** and/or **12B.** The pulse of the odour vapour being conveyed via lines **100A** and **100B** is joined in line **102** with the continuous flow air-line **100C** diluting it 1:3. Thus, for the 100 msec. pulse, 5 milliliters of air plus odour vapour is delivered, which represents 1.667 milliliters of odour vapour. In the case of reservoir **13A** containing amyl acetate, the concentration of amyl acetate emanating from reservoir **13A** through line **17A** and line **100A** is 518 micromoles/liter, or "518 μM"at 35°C. However the vapour temperature decreases to 24°C in line **101** prior to entry to line **102** where the concentration of the amyl acetate is 270 μM. Subsequent to dilution with air in line **102,** the concentration, resulting from 1:3 air dilution is 90 μM. When the air-vapour delivery rate is 3 liters per minute, the delivery rate of the amyl acetate is 270 μmoles/minute or 4.5 μmoles/second. Accordingly, 0.45 μmoles of amyl acetate is delivered in a 100 μsecond pulse.

During each experimental run, the four valves **12A, 12B, 15A** and **15B** are specially set by computer program. Airflow through the water reservoirs **14A, 14B** and **14C** is set to be continuous, but the airflow is diverted to pass through the odour reservoirs **13A,** containing the VA or amyl acetate and **13B** containing the ester mixture when required, for pulses of 100 microseconds duration and 10 second inter-stimulus interval. For each stimulus block 15 odour pulses are delivered.

Each experiment is divided into four sections. Each section is separated by a two-minute break. The four sections and their sequence are as follows:

(1) VA or amyl acetate (2%) alone;
(2) VA or amyl acetate (2%) plus binary ester mixture (20:80, 50:50, 80:20, 0.01% each ester mixture);
(3) VA or amyl acetate (2%) alone;
(4) Ester mixture alone.

## Subjects

Subjects are between 18-25 years old from the student population of the University and none has a history of olfactory dysfunction or respiratory disease. The protocol is explained to each subject and informed consent is obtained from each subject. During the experiment each of the subjects is seated in a comfortable chair in a test booth having a controlled environment. Each of the subjects is given a visual stimulus (10-cm TV playing silent cartoons) that maintains alertness (eliminated alpha-wave production) while minimizing eye movements and reducing blinking. Each of the subjects wears headphones through which white noise is played to eliminate auditory cues. Each of the subjects is given a button to press for the psychometric test so the subject could record odour pulse detection.

For the purpose of this experiment, electrodes are placed, as set forth in **Figure 2,** on the head, indicated by reference numeral **20** in **Figure 2,** of each subject in accordance with The International 10-20 System of Electrode Placement as explained in detail in Chapter 9, at pages 100-126, particularly page 110 of the text, James Hasset (1978) "A Primer of Psychophysiology". The International 10-20 System of Electrode Placement is the most widely used method to describe the location of scalp electrodes. The 10-20 system is based on the relationship between the location of an electrode and the underlying area of cerebral cortex. Each site is assigned a reference letter in order to facilitate identification of the lobe and a reference number or another reference letter to identify the hemisphere location.

As set forth in **Figure 2** and **Figure 3,** the reference letters used are:

"**F**" - Frontal lobe, the region of which is indicated by reference numeral **21;** "**T**"-Temporal lobe, the region of which is indicated by reference numeral **24 ;** "**C**" - Central lobe, "**P**" - Parietal lobe, the region of which is indicated by reference numeral **22; "O"-**Occipital lobe, the region of which is indicated by reference numeral **23.** It is herewith emphasized that there is no central lobe in the cerebral cortex. Accordingly, the reference letter "**C**" is used herein for identification purposes only. Even numbers affiliated with the reference letters to wit: **2**, **4**, **6** and **8** refer to the

right hemisphere, **33** and odd numbers affiliated with the reference letters in **Figure 2** and **Figure 3,** to wit:**1, 3, 5, 7** refer to the left hemisphere, **32.** The reference letter, "Z" refers to an electrode placed on the midline. The smaller the number, the closer the position to the midline. The reference,**"FP"** stands for "Front polar", the region of which is indicated by the references **FP1** and **FP2;** "Nasion" is the point between the forehead and nose, indicated by reference numeral **25.** "Inion" is the bump at the back portion, **34** of the skull, indicated by reference numeral **26**. The numerals "**10**" and "**20**" of the term "10-20 system" refer to the 10% and 20% interelectrode distance. Thus, as shown in **Figure 2** electrodes are placed at the following reference locations at the left side **A1** of each of the subjects, indicated by reference numeral **32: FP1, F3, C3, T3, P3** and **O1.** Correspondingly, electrodes are placed at the following reference locations at the right side **A2** of each of the subjects, indicated by reference numeral **33**: **FP2**, **F2**, **C2**, **T2** and **T6**.

## Recording and analysis of olfactory event-related potentials

Olfactory event-related potentials also referred to herein as OERP's are recorded using electroencephalography (EEG-5200, Nihon Kohden UK, Middlesex, UK). Electrodes are placed as set forth diagrammatically in **Figure 2,** with locations specified according to **Figure 3** according to the international 10/20 system, with reference to the side indicated by reference **A1** and reference numeral **32** and an earth electrode is placed on the forehead of each subject proximate the location indicated by reference numeral **31** in **Figure 3,** in the region of the location indicated by reference **FP1.** Data transmitted via electrode placed on each subject at reference numeral **CZ** are recorded and analysed in this study. Traces contaminated with eye movement artifacts detected via electrode located on each subject at reference numeral **FP1** are discarded. The time constant of the amplifiers is set to 0.5 seconds (≈1Hz high pass filter) and the data are low pass filtered at 35Hz. An additional 50Hz notch filter is used.

Analogue data were sent to a laboratory interface (CED1401), digitized at 100Hz and analyzed following signal averaging on a computer using "SIGNAL" analysis software (CED, Cambridge, UK). The "SIGNAL" analysis software and relevant use thereof is referred to at paragraphs 2.5 and 2.6 in Wang et al., "The correlation between physiological and psychological responses to odor stimulation in human subjects"Clinical Neurophysiology 113 (2002) 542-551.

The OERPs are measured from digitized records using the aforementioned SIGNAL analysis software (CED, Cambridge). In conformity with other studies of organoleptic event-related potentials, the OERP is taken to be the N1-$P_2/P_3$ waveform. The peak value or "amplitude" of the OERP is measured between manually set cursors. These are set just before $N_1$ and just after the $N_1$-$P_2/P_3$ waveform. Amplitude measurement could be semi-automated, removing subjectivity from the process. The noise, measured from the pre-stimulus baseline, was subtracted from this value.

## Psychometric tests

The psychometric tests are performed during the same experiment as the EEG recording. Subjects are presented with VA alone, VA in combination with ester mixture or ester mixture alone using the protocol described. Each of the subjects is requested to record the number of odour pulses which is detected by pressing a button connected to a laboratory interface. The timing and number of pulses is stored on the computer hard drive along with the EEG traces.

## Statistical analysis

Statistical differences of the data were evaluated by Student's t-test and considered significant at $P < 0.05$. Data were expressed as mean ± standard error of the mean (n = number of observation).

## Results

Referring to **Figure 4A, Figure 5A, Figure 6A, Figure 7A,** and **Figure 8A** the **"Y"** axis, represented by reference numerals **40, 50, 60, 70** and **80** provides the magnitude of the standardized organoleptic event related potential, "OERP" in each of **Figures 5A, 6A, 7A** and **8A,** respectively. In the data represented by **Figures 4A, 5A** and **6A,** n = 10. In the data represented by **Figure 7A,** n = 7.

In **Figure 4A,** showing the measurements of the counteraction of a valeric acid malodour by a 20:80 mixture of cyclohexylethan-1-yl butyrate and 1-cyclohexylethan-1-yl acetate, the bar graphs indicated by reference numerals **41** and **43** are for 2% n-valeric acid also termed herein,"VA". The bar graph indicated by reference numeral **42** is for a mixture of "VA" at a level of 2% and a 20:80 mixture of 1-cyclohexylethan-1-yl butyrate:1-cyclohexylethan-1-yl acetate at levels of 0.004% and 0.016%, respectively.

In **Figure 5A,** showing the measurements of the counteraction of a valeric acid malodour by a 50:50 mixture of cyclohexylethan-1-yl butyrate and 1-cyclohexylethan-1-yl acetate, the bar graphs indicated by reference numerals **51** and **53** are for 2% n-valeric acid also termed herein,"VA". The bar graph indicated by reference numeral **52** is for a

mixture of "VA" at a level of 2% and a 50:50 mixture of 1-cyclohexylethan-1-yl butyrate:1-cyclohexylethan-1-yl acetate at levels of 0.01% and 0.01%, respectively. The bar graph indicated by reference numeral **54** is for a 50:50 mixture of 1-cyclohexylethan-1-yl butyrate:1-cyclohexylethan-1-yl acetate at levels of 0.01% and 0.01%; respectively.

In **Figure 6A,** showing the measurements of the counteraction of a valeric acid malodour by an 80:20 mixture of cyclohexylethan-1-yl butyrate and 1-cyclohexylethan-1-yl acetate, the bar graphs indicated by reference numerals **61** and **63** are for 2% n-valeric acid also termed herein,"VA". The bar graph indicated by reference numeral **62** is for a mixture of "VA" at a level of 2% and an 80:20 mixture of 1-cyclohexylethan-1-yl butyrate:1-cyclohexylethan-1-yl acetate at levels of 0.016% and 0.004%, respectively.

In **Figure 7A,** showing the measurements of the counteraction of an amyl acetate pleasant aroma by a 20:80 mixture of cyclohexylethan-1-yl butyrate and 1-cyclohexylethan-1-yl acetate, the bar graphs indicated by reference numerals **71** and **73** are for 2% amyl acetate. The bar graph indicated by reference numeral **72** is for a mixture of amyl acetate at a level of 2% and a 20:80 mixture of 1-cyclohexylethan-1-yl butyrate: 1-cyclohexylethan-1-yl acetate at levels of 0.004% and 0.016%, respectively.

In **Figure 8A,** showing the comparison of the % inhibition of OERP for ester mixtures of our invention vs. the individual components of the ester mixtures of our invention, the bar graph indicated by reference numeral **81** is for an 80:20 mixture of 1-cyclohexylethan-1-yl butyrate:1-cyclohexylethan-1-yl acetate; the bar graph indicated by reference numeral **82** is for a 50:50 mixture of 1-cyclohexylethan-1-yl butyrate: 1-cyclohexylethan-1-yl acetate; the bar graph indicated by reference numeral **83** is for a 20:80 mixture of 1-cyclohexylethan-1-yl butyrate:1-cyclohexylethan-1-yl acetate; the bar graph indicated by reference numeral **84** is for 1-cyclohexylethan-1-yl butyrate, alone; and the bar graph indicated by reference numeral **85** is for 1-cyclohexylethan-1-yl acetate, alone. Reference numeral **85a** indicates the standard deviation for the bar graph indicated by reference numeral **85**.

Referring to **Figure 4B, Figure 5B, Figure 6B** and **Figure 7B** the "Y" axis, represented by reference numerals **44, 55, 64** and **74** provides the magnitude of the % psychoinetric-cognitive recognition of odour or malodour as the case may be, also referred to as "% perception" in each of **Figures 5B**, **6B** and **7B** respectively. In the data represented by **Figures 4B, 5B** and **6B,** n = 10. In the data represented by **Figure 7B,** n = 7. In **Figure 8B,** the "Y" axis, represented by reference numeral **86** provides the magnitude of the % inhibition of perception.

In **Figure 4B,** showing the measurements of the counteraction of a valeric acid malodour by a 20:80 mixture of cyclohexylethan-1-yl butyrate and 1-cyclohexylethan-1-yl acetate, the bar graphs indicated by reference numerals **45** and **47** are for 2% n-valeric acid also termed herein,"VA". The bar graph indicated by reference numeral **46** is for a mixture of "VA" at a level of 2% and a 20:80 mixture of 1-cyclohexylethan-1-yl butyrate:l-cyclohexylethan-1-yl acetate at levels of 0.004% and 0.016%, respectively. Reference numeral **47a** indicates the standard deviation for the bar graph indicated by reference numeral **47**.

In **Figure 5B,** showing the measurements of the counteraction of a valeric acid malodour by a 50:50 mixture of cyclohexylethan-1-yl butyrate and 1-cyclohexylethan-1-yl acetate, the bar graphs indicated by reference numerals **56** and **58** are for 2% n-valeric acid also termed herein,"VA". The bar graph indicated by reference numeral **57** is for a mixture of "VA" at a level of 2% and a 50:50 mixture of 1-cyclohexylethan-1-yl butyrate:1-cyclohexylethan-1-yl acetate at levels of 0.01% and 0.01%, respectively. The bar graph indicated by reference numeral **59** is for a 50:50 mixture of 1-cyclohexylethan-1-yl butyrate:1-cyclohexylethan-1-yl acetate at levels of 0.01% and 0.01%, respectively. Reference numeral **59a** indicates the standard deviation for the bar graph indicated by reference numeral **59.**

In **Figure 6B,** showing the measurements of the counteraction of a valeric acid malodour by an 80:20 mixture of cyclohexylethan-1-yl butyrate and 1-cyclohexylethan-l-yl acetate, the bar graphs indicated by reference numerals **65** and **67** are for 2% n-valeric acid also termed herein,"VA". The bar graph indicated by reference numeral **66** is for a mixture of "VA" at a level of 2% and an 80:20 mixture of 1-cyclohexylethan-1-yl butyrate:l-cyclohexylethan-1-yl acetate at levels of 0.016% and 0.004%, respectively. Reference numeral **67a** indicates the standard deviation for the bar graph indicated by reference numeral **67**.

In **Figure 7B,** showing the measurements of the counteraction of an amyl acetate pleasant aroma by a 20:80 mixture of cyclohexylethan-1-yl butyrate and 1-cyclohexylethan-1-yl acetate, the bar graphs indicated by reference numerals **75** and **77** are for 2% amyl acetate. The bar graph indicated by reference numeral **72** is for a mixture of amyl acetate at a level of 2% and a 20:80 mixture of 1-cyclohexylethan-1-yl butyrate:1-cyclohexylethaa-1-yl acetate at levels of 0.00496 and 0.016%, respectively. Reference numeral **77a** indicates the standard deviation for the bar graph indicated by reference numeral **77.**

In **Figure 8B** showing the comparison of the % inhibition of perception for ester mixtures of our invention vs. the individual components of the ester mixtures of our invention, the bar graph indicated by reference numeral **87** is for an 80:20 mixture of 1-cyclohexylethan-1-yl butyrate:1-eyclohexylethan-1-yl acetate; the bar graph indicated by reference numeral **88** is for a 50:50 mixture of 1-cyclohexylethan-1-yl butyrate:1-cyclohexylethan-1-yl acetate; the bar graph indicated by reference numeral **89** is for a 20:80 mixture of 1-cyclohexylethan-1-yl butyrate:1-cyclohexylethan-1-yl acetate; the bar graph indicated by reference numeral **841** is for 1-cyclohexylethan-1-yl butyrate, alone; and the bar graph indicated by reference numeral **851** is for 1-cyclohexylethan-1-yl acetate, alone.

## Conclusion

As a result of the ester mixtures being evaluated in the proportions; 20:80, 50:50 and 80:20 as set forth in **Figures 4-8,** inclusive, whereby the results of the physiological and psychometric tests were compared the following conclusions are herewith made:

(a) The ester mixtures in the ratios of 20:80, 50:50 and 80:20 did not counteract a control odour as confirmed by the results summarized in **Figure 7A** and **Figure 7B.**

(b) All combinations of the ester mixtures caused significant counteraction of valeric acid in the ratios of 20.80, 50:50 and 80:20 in both physiological and psychometric testing as confirmed by the results summarized in **Figures 4A, 4B, 5A, 5B, 6A, 6B, 8A and 8B.**

(c) Performing an independent samples two-tailed t-test on the data demonstrated that:

(i) The 20:80 weight ratio 1-cyclohexytethan-1-yl butyrate: 1-cyclohexylethan-1-yl acetate mixture performed unexpectedly and significantly better than either 1-cyelohexylethan-1-yl butyrate or 1-eyclohexylethan-1-yl acetate alone (p = 0.027 and 0.022, respectively) on the basis of the physiological test; and
(ii) The 50:50 weight ratiol-cyclohexylethan-1-yl butyrate: 1-cyclohexylethan-1-yl acetate mixture performed better than the 1-clohexylethan-1-yl butyrate and the 1-cyclohexylethan-1-yl acetate alone on the basis of the physiological test (p = 0.099 and 0.49, respectively).

## DETAILED DESCRIPTION OF FIGURES 9A, 9B, 9C and 9D

**Figures 9A, 9B, 9C** and **9D** are herein utilized to aid in interpreting the meanings of the parameters: $\mathbf{Z_o}$, **A, B, C** and **D** in the model:

$$\mathbf{Z = Z_o + A(1\text{-}B^X) + C(1\text{-}D^Y)}$$

wherein **Z** is the percentage of odour pulses correctly identified;
**Y** is the pulse duration, or "pulse length" measured in units of microseconds; and
X is the inter-stimulus interval, "ISI", measured in units of seconds.

**Figure 9A** is a dose-stimulus-frequency set of bar graphs for the malodour, n-butyric acid shown in three dimensions using the "X" axis, indicated by reference numeral **92A,** the "Y" axis, indicated by reference numeral **91A** and the "Z" axis, indicated by reference numeral **90A.** The set of bar graphs indicated by reference numeral **97A** is for a pulse length of 35 microseconds. The set of bar graphs indicated by reference numeral **96A** is for a pulse length of 50 microseconds. The set of bar graphs indicated by reference numeral **95A** is for a pulse length of 75 microseconds. The set of bar graphs indicated by reference numeral **94A** is for a pulse length of 100 microseconds. The set of bar graphs indicated by reference numeral **93A** is for a pulse length of 200 microseconds. The curved surface setting forth the relationship of the variables, X, Y and Z is indicated by reference numeral **98A**.

**Figure 9B** is a dose-stimulus-frequency set of bar graphs for the "pleasant" odour, amyl acetate, shown in three dimensions using the "X" axis, indicated by reference numeral **92B,** the "Y" axis, indicated by reference numeral **91B** and the "Z" axis, indicated by reference numeral **90B**. The set of bar graphs indicated by reference numeral **97B** is for a pulse length of 35 microseconds. The set of bar graphs indicated by reference numeral **96B** is for a pulse length of 50 microseconds. The set of bar graphs indicated by reference numeral **95B** is for a pulse length of 75 microseconds. The set of bar graphs indicated by reference numeral **94B** is for a pulse length of 100 microseconds. The set of bar graphs indicated by reference numeral **93B** is for a pulse length of 200 microseconds. The curved surface setting forth the relationship of the variables, **X**, **Y** and **Z** is indicated by reference numeral **98B.**

**Figure 9C** is a set of adaptation curves for the malodour, n-butyric acid, with the vertical axis being the "Z' axis, indicated by reference numeral **90C** and the horizontal axis being the "X" axis indicated by reference numeral **92C**. The graph indicated by reference numeral **97C** and data points therefor **970C** is for a pulse length of 35 microseconds. The graph indicated by reference numeral **96C** and data points therefor **960C** is for a pulse length of 50 microseconds. The graph indicated by reference numeral **95C** and data points therefor **950C** is for a pulse length of 75 microseconds. The graph indicated by reference numeral **94C** and data points therefor **940C** is for a pulse length of 100 microseconds. The graph indicated by reference numeral **93C** and data points therefor **930C** is for a pulse length of 200 microseconds.

**Figure 9D** is a set of adaptation curves for the "pleasant" odour, amyl acetate, with the vertical axis being the "Z' axis, indicated by reference numeral **90D** and the horizontal axis being the "X" axis indicated by reference numeral **92D.** The graph indicated by reference numeral **97D** and data points therefor **970D** is for a pulse length of 35 microseconds. The graph indicated by reference numeral **96D** and data points therefor **960D** is for a pulse length of 50 microseconds. The graph indicated by reference numeral **95D** and data points therefor **950D** is for a pulse length of 75 microseconds. The graph indicated by reference numeral **94D** and data points therefor **940D** is for a pulse length of 100 microseconds. The graph indicated by reference numeral **93D** and data points therefor **930**D is for a pulse length of 200 microseconds.

In relating the substance of **Figures 9A** and **9B,** to the model:

$$Z = Z_o + A(1\text{-}B^X) + C(1\text{-}D^Y)$$

the parameters **C** and **D** are measures of the effects of (i) pulse duration , **Y** measured in microseconds, "ms", along the "Y" axes indicated by reference numerals **91A** and **91B** and (ii) interstimulus interval, "ISI" measured in seconds, "s", along the "X" axes indicated by reference numerals **92A** and **92B** in **Figures 9A** and **9B,** respectively , on (iii) the percentage of odour pulses correctly identified, **Z,** measured along the "Z" axes indicated by reference numerals **90A** and **90B** in **Figures 9A** and **9B,** respectively and each of **C** and **D** is a measure of the angle of inclination,θ, of the normal to the tangent plane to the **X-Y-Z** surface, indicated by reference numerals **98A** and **98B** in **Figures 9A** and **9B,** respectively at a point $(X_1,Y_1,Z_1)$ wherein $\theta = \arccos[|\nabla F\ (X_1,Y_1,Z_1).|k\ |/\{\|\nabla F\ (X_1,Y_1,Z_1)\|\}]$.

In relating the substance of **Figures 9C** and **9D,** to the model:

$$Z = Z_o + A(1\text{-}B^X) + C(1\text{-}D^Y)$$

concerning the **A** and **B** parameters, each parameter defines the effects of the inter-stimulus interval, **X,** measured along the "X" axis indicated by reference numerals **92C** and **92D,** respectively for **Figures 9C** and **9D,** and stimulus frequency, ϕ, on the percentage of odour pulses correctly identified, Z, measured along the vertical axes **90C** and **90D** in **Figures 9C** and **9D,** respectively, and each is a measure of the degree of adaptation to malodours and habituation as measured by the tangent slopes, **99C** and **99D** to **X-Z** curves **93C** and **93D** at given points, **990C** ($X_1$ = 12, $Z_1$ = 75), $\nabla$ F($X_1$, $Z_1$) in **Figure 9C** and **990D** ($X_1$ = 15, $Z_1$ = 54), $\nabla$ F($X_1$, $Z_1$) in **Figure 9D.** Also, **C** is a concentration parameter and is a measure of the magnitude of the tangent slope to the **Y-Z** curve at a given point, ($Y_1$, $Z_1$), $\nabla$ F($Y_1$, $Z_1$).

## Claims

1. A mixture consisting essentially of 1-cyclohexylethan-1-yl butyrate having the structure:

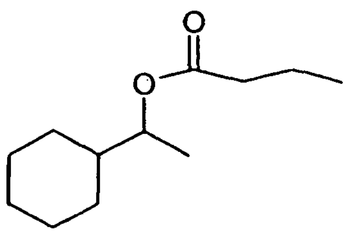

and 1-cyclohexylethan-1-yl acetate having the structure:

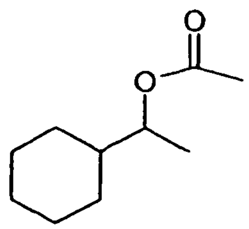

the weight ratio of 1-cyclohexylethan-1-yl butyrate:1-cyclohexylethan-1-yl acetate being from 20:80 up to 50:50, in the substantial absence of the compounds: 1-cyclohexylethan-1-ol having the structure:

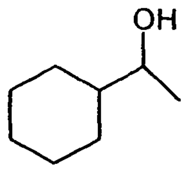

1-(4'-methylethyl)cyclohexylethan-1-yl propionate having the structure:

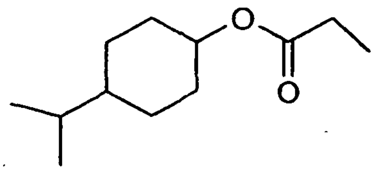

and 2'-hydroxy-1'-ethyl(2-phenoxy)acetate having the structure:

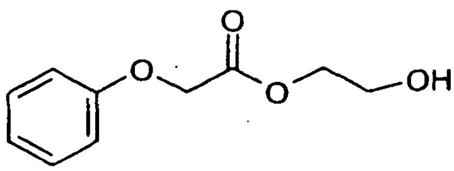

2. A process for counteracting a malodour present in a defined air 3-dimensional volume, as measured using "x", "y" and "z" coordinates, comprising the step of introducing into said defined air 3-dimensional volume, as measured using "x", "y" and "z" coordinates, a malodour counteracting quantity and concentration of the composition of claim 1 as a single dose, as a continuous dose over a malodour-counteracting period of time, or as periodic doses over a malodour-counteracting period of time

3. A process according to claim 2 wherein the defined air 3-dimensional volume, as measured using "x", "y" and "z" coordinates, is fragrance-containing and whereby the perceived odor intensity of the fragrance existant in said defined air 3-dimensional volume, as measured using "x", "y" and "z" coordinates, is substantially maintained.

4. The process of claim 3 wherein the malodour eminates from a solid or liquid malodourous source, proximate said 3-dimensional volume, into said 3-dimensional volume and said solid or liquid malodourous source evolving the malodour is selected from the group consisting of :

i. a malodourous herbicide;
ii. a malodourous antiviral composition;
iii. a malodourous fungicide;

iv. a malodourous bactericide;
v. a malodourous parasiticide;
vi. a malodourous insecticide;
vii. a malodourous depilatory preparation;
viii. a malodourous bleach composition;
ix. a malodourous hard surface-cleaning preparation;
x. a malodourous skin cleansing composition;
xi. a malodourous anti-microbial nail preparation;
xii. a malodourous hair setting composition;
xiii. a malodourous hair conditioning composition;
xiv. a malodourous trichological lotion;
xv. a malodourous skin lightening composition;
xvi. a malodourous detergent composition;
xvii. a malodourous soap composition;
xviii. a malodourous sunscreen composition;
xix. a malodourous fabric stain removal composition;
xx. a malodourous fabric conditioning composition;
xxi. a malodourous fabric anti-wrinkle composition;
xxii. a malodourous steam iron aroma composition;
xxiii. a malodourous candle composition;
xxiv. a malodourous plant growth regulating composition;
xxv. a malodourous plant growth stimulating composition;
xxvi. a malodourous fertilizer composition;
xxvii. a malodourous insect attractant composition;
xxviii. a malodourous insect repelling composition;
xxix. a malodourous drain cleaning composition, and
xxx. a malodourous molluskicide composition;

**5.** The process of claim 4 wherein the malodour is caused by a malodour-causing quantity and concentration of at least one compound selected from the group consisting of aliphatic halohydrins, aliphatic amines, aliphatic N-oxides, dialkylamines, cycloaliphatic amines, cycloaliphatic N-oxides, cyclo-olefinic amines, cyclo-olefinic N-oxides, cycloaromatic amines, cycloaromatic N-oxides, hydroxyalkylamines, imine compounds, amide compounds, amino acids, polypeptides, modified antimicrobial proteins, diureides, nitriles, aliphatic mercaptans, cycloaliphatic mercaptans, mercaptoalkanoic acids, mercaptoalkanoic acid esters, aliphatic mono sulfides, disulfides, trisulfides, sulfur oxides, sulfones and sultones, cycloaliphatic monosulfides, disulfides, trisulfides, sulfur oxides, sulfones and sultones, cyclo-olefinic monosulfides, disulfides, trisulfides, sulfur oxides, sulfones and sultones, cycloaromatic monosulfides, disulfides, trisulfides, sulfur oxides, sulfones and sultones, isothiocyanates, thiocyanates, dithiocyanates, isothiazolones, isothiazolinones, thiodiazinethiones, halosulfamates, aryl sulfonamides, lower aliphatic carboxylic acids, phenols, phosphines, aliphatic phosphites and phosphonates, cycloaliphatic phosphites and phosphonates, arsines, lower alcohols, lower ketones, hops, hops acids, aryl pyrazoles, oxazolines, isocyanurates, biguanides, extracts of krameria, hydantoins, pyrollidones, pyrollidone carboxylic acids, pyrollidone carboxylic acid esters, nitrophenols, N-substituted aspartic acids and pyrethroids.

**6.** A method for counteracting a malodour in a solid or liquid fragrance-containing soap or detergent caused by a malodour-causing quantity and concentration of at least one compound selected from the group consisting of aliphatic halohydrins, aliphatic amines, aliphatic N-oxides, dialkylamines, cycloaliphatic amines, cycloaliphatic N-oxides, cyclo-olefinic amines, cyclo-olefinic N-oxides, cycloaromatic amines, cycloaromatic N-oxides, hydroxyalkylamines, imine compounds, amide compounds, amino acids, polypeptides, modified antimicrobial proteins, diureides, nitriles, aliphatic mercaptans, cycloaliphatic mercaptans, mercaptoalkanoic acids, mercaptoalkanoic acid esters, aliphatic monosulfides, disulfides, trisulfides, sulfur oxides, sulfones and sultones, cycloaliphatic monosulfides, disulfides, trisulfides, sulfur oxides, sulfones and sultones, cyclo-olefinic monosulfides, disulfides, trisulfides, sulfur oxides, sulfones and sultones, cycloaromatic monosulfides, disulfides, trisulfides, sulfur oxides, sulfones and sultones, isothiocyanates, thiocyanates, dithiocyanates, isothiazolones, isothiazolinones, thiodiazinethiones, halosulfamates, aryl sulfonamides, lower aliphatic carboxylic acids, phenols, phosphines, aliphatic phosphites and phosphonates, cycloaliphatic phosphites and phosphonates, arsines, lower alcohols, lower ketones, hops, hops acids, aryl pyrazoles, oxazolines, isocyanurates, biguanides, extracts of krameria, hydantoins, pyrollidones, pyrollidone carboxylic acids, pyrollidone carboxylic acid esters, nitrophenols, N-substituted aspartic acids and pyrethroids comprising the step of introducing into the solid or liquid soap or detergent an effective malodour counteracting quantity and concentration of the composition of claim 1 whereby the perceived odor intensity of the fragrance contained in the solid or liquid soap or detergent is substantially maintained.

**7.** A method of counteracting a malodour in a fragranced air 3-dimensional volume, as measured using "x", "y" and "z" coordinates, caused by a malodour-causing quantity and concentration of at least one compound selected from the group consisting of aliphatic halohydrins, aliphatic amines, aliphatic N-oxides, dialkylamines, cycloaliphatic amines, cycloaliphatic N-oxides, cyclo-olefinic amines, cyclo-olefinic N-oxides, cycloaromatic amines, cycloaromatic N-oxides, hydroxyalkylamines, imine compounds, amide compounds, amino acids, polypeptides, modified antimicrobial proteins, diureides, nitriles, aliphatic mercaptans, cycloaliphatic mercaptans, mercaptoalkanoic acids, mercaptoalkanoic acid esters, aliphatic monosulfides, disulfides, trisulfides, sulfur oxides, sulfones and sultones, cycloaliphatic monosulfides, disulfides, trisulfides, sulfur oxides, sulfones and sultones, cyclo-olefinic monosulfides, disulfides, trisulfides, sulfur oxides, sulfones and sultones, cycloaromatic monosulfides, disulfides, trisulfides, sulfur oxides, sulfones and sultones, isothiocyanates, thiocyanates, dithiocyanates, isothiazolones, isothiazolinones, thiodiazinethiones, halosulfamates, aryl sulfonamides, lower aliphatic carboxylic acids, phenols, phosphines, aliphatic phosphites and phosphonates, cycloaliphatic phosphites and phosphonates, arsines, lower alcohols, lower ketones, hops, hops acids, aryl pyrazoles, oxazolines, isocyanurates, biguanides, extracts of krameria, hydantoins, pyrollidones, pyrollidone carboxylic acids, pyrollidone carboxylic acid esters, nitrophenols, N-substuted aspartic acids and pyrethroids comprising the step of introducing into said fragranced air 3-dimensional volume, as measured using "x", "y" and "z" coordinates, an effective malodour counteracting quantity and concentration of the composition of claim 1 whereby the perceived odor intensity of the fragrance contained in the fragranced air 3-dimensional volume, as measured using "x", "y" and "z" coordinates, is substantially maintained.

**Patentansprüche**

**1.** Gemisch, im Wesentlichen bestehend aus 1-Cyclohexylethan-1-ylbutyrat mit der Struktur

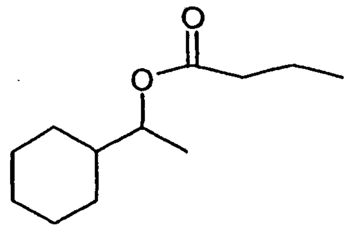

und 1-Cyclohexylethan-1-ylacetat mit der Struktur

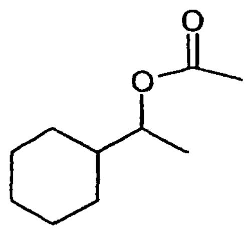

wobei das Gewichtsverhältnis von 1-Cyclohexylethan-1-ylbutyrat:1-Cyclohexylethan-1-ylacetat von 20:80 bis 50:50 beträgt,
in der im Wesentlichen Abwesenheit der Verbindungen 1-Cyclohexylethan-1-ol mit der Struktur

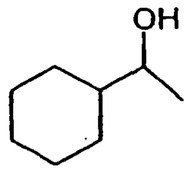

1-(4'-Methylethyl)cyclohexylethanl-ylpropionat mit der Struktur

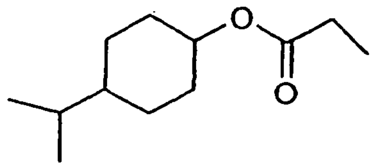

und 2'-Hydroxy-1'-ethyl(2-phenoxy)acetat mit der Struktur

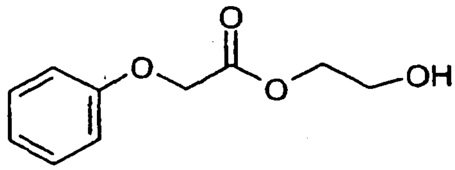

2. Verfahren, um einem schlechten Geruch, der in einem definierten 3-dimensionalen Luftvolumen, wie unter Verwendung von "x"-, "y"- und "z"-Koordinaten gemessen, vorliegt, entgegenzuwirken, umfassend den Schritt des Einführens einer schlechtem Geruch entgegenwirkenden Menge und Konzentration der Zusammensetzung nach Anspruch 1 als eine Einzeldosis, als eine kontinuierliche Dosis über einen Zeitraum, während dessen schlechtem Geruch entgegengewirkt wird, oder als periodische Dosen über einen Zeitraum, während dessen schlechtem Geruch entgegengewirkt wird, in das definierte 3-dimensionale Luftvolumen, wie unter Verwendung von "x"-, "y"- und "z"-Koordinaten gemessen.

3. Verfahren nach Anspruch 2, wobei das definierte 3-dimensionale Luftvolumen, wie unter Verwendung von "x"-, "y"- und "z"-Koordinaten gemessen, Duft-enthaltend ist, und wodurch die wahrgenommene Geruchsintensität des Dufts, der in dem definierten 3-dimensionalen Luftvolumen, wie unter Verwendung von "x"-, "y"- und "z"-Koordinaten gemessen, vorhanden ist, im Wesentlichen beibehalten wird.

4. Verfahren nach Anspruch 3, wobei der schlechte Geruch von einer festen oder flüssigen schlecht riechenden Quelle in der Nähe des dreidimensionalen Volumens in das dreidimensionale Volumen strömt und die feste oder flüssige schlecht riechende Quelle, die den schlechten Geruch entwickelt, aus der Gruppe, bestehend aus

i. einem schlecht riechenden Herbizid,
ii. einer schlecht riechenden antiviralen Zusammensetzung,
iii. einem schlecht riechenden Fungizid,
iv. einem schlecht riechenden Bakterizid,
v. einem schlecht riechenden Parasitizid,
vi. einem schlecht riechenden Insektizid,
vii. einer schlecht riechenden Enthaarungsmittelzubereitung,
viii. einer schlecht riechenden Bleichmittelzusammensetzung,
ix. einer biologisch nicht abbaubaren Reinigungsmittelzubereitung mit schlechten Geruch,
x. einer schlecht riechenden Hautreinigungszusammensetzung,

xi. einer schlecht riechenden antimikrobiellen Nagelzubereitung,
xii. einer schlecht riechenden Haarfestigerzusammensetzung,
xiii. einer schlecht riechenden Haarpflegezusammensetzung,
xiv. einer schlecht riechenden haarmedizinischen Lotion,
xv. einer schlecht riechenden Hautaufhellungszusammensetzung,
xvi. einer schlecht riechenden Waschmittelzusammensetzung,
xvii. einer schlecht riechenden Seifenzusammensetzung,
xviii. einer schlecht riechenden Sonnenschutzmittelzusammensetzung,
xix. einer schlecht riechenden Gewebefleckenentfernungszusammensetzung,
xx. einer schlecht riechenden Gewebepflegezusammensetzung,
xxi. einer schlecht riechenden Gewebeantiknitterzusammensetzung,
xxii. einer schlecht riechenden Dampfbügeleisenduftzusammensetzung,
xxiii. einer schlecht riechenden Kerzenzusammensetzung,
xxiv. einer schlecht riechenden Pflanzenwachstumsregulierungszusammensetzung,
xxv. einer schlecht riechenden Pflanzenwachstumsstimulierungszusammensetzung,
xxvi. einer schlecht riechenden Düngemittelzusammensetzung,
xxvii. einer schlecht riechenden Insektenanziehungszusammensetzung,
xxviii. einer schlecht riechenden Insektenabwehrzusammensetzung,
xxix. einer schlecht riechenden Abflußreinigungszusammensetzung und
xxx. einer schlecht riechenden Molluskizidzusammensetzung ausgewählt ist.

**5.** Verfahren nach Anspruch 4, wobei der schlechte Geruch durch eine schlechten Geruch verursachende Menge und Konzentration mindestens einer Verbindung, ausgewählt aus der Gruppe, bestehend aus aliphatischen Halogenhydrinen, aliphatischen Aminen, aliphatischen N-Oxiden, Dialkylaminen, cycloaliphatischen Aminen, cycloaliphatischen N-Oxiden, cyclo-olefinischen Aminen, cyclo-olefinischen N-Oxiden, cycloaromatischen Aminen, cycloaromatischen N-Oxiden, Hydroxyalkylaminen, Iminverbindungen, Amidverbindungen, Aminosäuren, Polypeptiden, modifizierten antimikrobiellen Proteinen, Diureiden, Nitrilen, aliphatischen Mercaptanen, cycloaliphatischen Mercaptanen, Mercaptoalkansäuren, Mercaptoalkansäureestern, aliphatischen Monosulfiden, Disulfiden, Trisulfiden, Schwefeloxiden, Sulfonen und Sultonen, cycloaliphatischen Monosulfiden, Disulfiden, Trisulfiden, Schwefeloxiden, Sulfonen und Sultonen, cyclo-olefinischen Monosulfiden, Disulfiden, Trisulfiden, Schwefeloxiden, Sulfonen und Sultonen, cycloaromatischen Monosulfiden, Disulfiden, Trisulfiden, Schwefeloxiden, Sulfonen und Sultonen, Isothiocyanaten, Thiocyanaten, Dithiocyanaten, Isothiazolonen, Isothiazolinonen, Thiodiazinthionen, Halogensulfamaten, Arylsulfonamiden, niederen aliphatischen Carbonsäuren, Phenolen, Phosphinen, aliphatischen Phosphiten und Phosphonaten, cycloaliphatischen Phosphiten und Phosphonaten, Arsinen, niederen Alkoholen, niederen Ketonen, Hopfen, Hopfensäuren, Arylpyrazolen, Oxazolinen, Isocyanuraten, Biguaniden, Krameriaextrakten, Hydantoinen, Pyrollidonen, Pyrollidoncarbonsäuren, Pyrollidoncarbonsäureestern, Nitrophenolen, N-substituierten Asparaginsäuren und Pyrethroiden, verursacht wird.

**6.** Verfahren, um einem schlechten Geruch in einer festen oder flüssigen Duftenthaltenden Seife oder Reinigungsmittel entgegenzuwirken, verursacht durch eine schlechten Geruch verursachende Menge und Konzentration mindestens einer Verbindung, ausgewählt aus der Gruppe, bestehend aus aliphatischen Halogenhydrinen, aliphatischen Aminen, aliphatischen N-Oxiden, Dialkylaminen, cycloaliphatischen Aminen, cycloaliphatischen N-Oxiden, cyclo-olefinischen Aminen, cyclo-olefinischen N-Oxiden, cycloaromatischen Aminen, cycloaromatischen N-Oxiden, Hydroxyalkylaminen, Iminverbindungen, Amidverbindungen, Aminosäuren, Polypeptiden, modifizierten antimikrobiellen Proteinen, Diureiden, Nitrilen, aliphatischen Mercaptanen, cycloaliphatischen Mercaptanen, Mercaptoalkansäuren, Mercaptoalkansäureestern, aliphatischen Monosulfiden, Disulfiden, Trisulfiden, Schwefeloxiden, Sulfonen und Sultonen, cycloaliphatischen Monosulfiden, Disulfiden, Trisulfiden, Schwefeloxiden, Sulfonen und Sultonen, cyclo-olefinischen Monosulfiden, Disulfiden, Trisulfiden, Schwefeloxiden, Sulfonen und Sultonen, cycloaromatischen Monosulfiden, Disulfiden, Trisulfiden, Schwefeloxiden, Sulfonen und Sultonen, Isothiocyanaten, Thiocyanaten, Dithiocyanaten, Isothiazolonen, Isothiazolinonen, Thiodiazinthionen, Halogensulfamaten, Arylsulfonamiden, niederen aliphatischen Carbonsäuren, Phenolen, Phosphinen, aliphatischen Phosphiten und Phosphonaten, cycloaliphatischen Phosphiten und Phosphonaten, Arsinen, niederen Alkoholen, niederen Ketonen, Hopfen, Hopfensäuren, Arylpyrazolen, Oxazolinen, Isocyanuraten, Biguaniden, Krameriaextrakten, Hydantoinen, Pyrollidonen, Pyrollidoncarbonsäuren, Pyrollidoncarbonsäureestern, Nitrophenolen, N-substituierten Asparaginsäuren und Pyrethroiden, umfassend den Schritt des Einführens einer schlechtem Geruch entgegenwirkenden wirksamen Menge und Konzentration der Zusammensetzung nach Anspruch 1 in die feste oder flüssige Seife oder Reinigungsmittel, wodurch die wahrgenommene Geruchsintensität des in der festen oder flüssigen Seife oder Reinigungsmittel enthaltenen Dufts im Wesentlichen beibehalten wird.

7. Verfahren, um einem schlechten Geruch, der in einem mit Duft versehenen 3-dimensionalen Luftvolumen, wie unter Verwendung von "x"-, "y"- und "z"-Koordinaten gemessen, vorliegt, entgegenzuwirken, verursacht durch eine schlechten Geruch verursachende Menge und Konzentration mindestens einer Verbindung, ausgewählt aus der Gruppe, bestehend aus aliphatischen Halogenhydrinen, aliphatischen Aminen, aliphatischen N-Oxiden, Dialkylaminen, cycloaliphatischen Aminen, cycloaliphatischen N-Oxiden, cyclo-olefinischen Aminen, cyclo-olefinischen N-Oxiden, cycloaromatischen Aminen, cycloaromatischen N-Oxiden, Hydroxyalkylaminen, Iminverbindungen, Amidverbindungen, Aminosäuren, Polypeptiden, modifizierten antimikrobiellen Proteinen, Diureiden, Nitrilen, aliphatischen Mercaptanen, cycloaliphatischen Mercaptanen, Mercaptoalkansäuren, Mercaptoalkansäureestern, aliphatischen Monosulfiden, Disulfiden, Trisulfiden, Schwefeloxiden, Sulfonen und Sultonen, cycloaliphatischen Monosulfiden, Disulfiden, Trisulfiden, Schwefeloxiden, Sulfonen und Sultonen, cyclo-olefinischen Monosulfiden, Disulfiden, Trisulfiden, Schwefeloxiden, Sulfonen und Sultonen, cycloaromatischen Monosulfiden, Disulfiden, Trisulfiden, Schwefeloxiden, Sulfonen und Sultonen, Isothiocyanaten, Thiocyanaten, Dithiocyanaten, Isothiazolonen, Isothiazolinonen, Thiodiazinthionen, Halogensulfamaten, Arylsulfonamiden, niederen aliphatischen Carbonsäuren, Phenolen, Phosphinen, aliphatischen Phosphiten und Phosphonaten, cycloaliphatischen Phosphiten und Phosphonaten, Arsinen, niederen Alkoholen, niederen Ketonen, Hopfen, Hopfensäuren, Arylpyrazolen, Oxazolinen, Isocyanuraten, Biguaniden, Krameriaextrakten, Hydantoinen, Pyrollidonen, Pyrollidoncarbonsäuren, Pyrollidoncarbonsäureestern, Nitrophenolen, N-substituierten Asparaginsäuren und Pyrethroiden, umfassend den Schritt des Einführens einer schlechtem Geruch entgegenwirkenden wirksamen Menge und Konzentration der Zusammensetzung nach Anspruch 1 in das mit Duft versehene 3-dimensionale Luftvolumen, wie unter Verwendung von "x"-, "y"- und "z"-Koordinaten gemessen, wodurch die wahrgenommene Geruchsintensität des Dufts, der in dem mit Duft versehenen 3-dimensionalen Luftvolumen, wie unter Verwendung von "x"-, "y"- und "z"-Koordinaten gemessen, vorhanden ist, im Wesentlichen beibehalten wird.

## Revendications

1. Mélange constitué essentiellement de butyrate de 1-cyclohexyléthan-1-yle, ayant la structure :

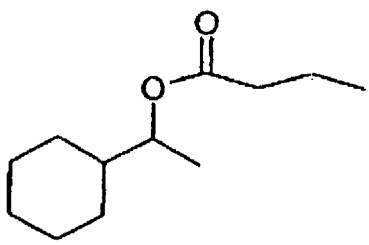

et d'acétate de 1-cyclohexyléthan-1-yle, ayant la structure :

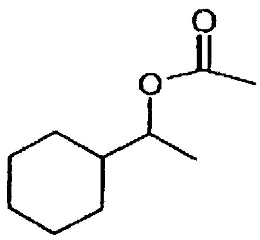

le rapport pondéral du butyrate de 1-cyclohexyléthan-1-yle à l'acétate de 1-cyclohexyléthan-1-yle étant de 20:80 à 50:50, sensiblement en l'absence des composés suivants :
1-cyclohexyléthan-1-ol, ayant la structure :

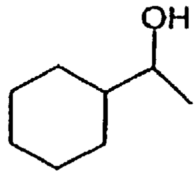

propionate de 1-(4'-méthyléthyl)cyclohexyléthan-1-yle, ayant la structure :

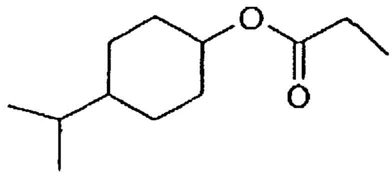

et (2-phénoxy)-acétate de 2'-hydroxy- 1'-éthyle, ayant la structure :

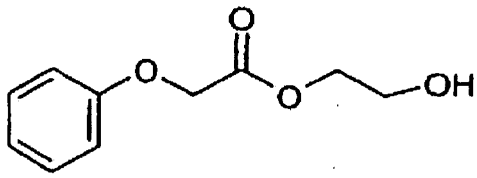

**2.** Procédé pour contrecarrer une mauvaise odeur présente dans un volume d'air tridimensionnel défini, tel que mesuré en utilisant les coordonnées "x", "y" et "z", comprenant l'étape consistant à introduire dans ledit volume d'air tridimensionnel défini, tel que mesuré en utilisant les coordonnées "x", "y" et "z", une quantité et une concentration contrecarrant la mauvaise odeur de la composition selon la revendication 1 sous forme d'une dose unique, sous forme d'une dose continue pendant une période de temps contrecarrant la mauvaise odeur, ou sous forme de doses périodiques pendant une période de temps contrecarrant la mauvaise odeur.

**3.** Procédé selon la revendication 2, dans lequel le volume d'air tridimensionnel défini, tel que mesuré en utilisant les coordonnées "x", "y" et "z" contient une fragrance, et dans lequel l'intensité d'odeur perçue de la fragrance existant dans ledit volume d'air tridimensionnel défini, tel que mesuré en utilisant les coordonnées "x", "y" et "z", est sensiblement maintenue.

**4.** Procédé selon la revendication 3, dans lequel la mauvaise odeur se dégage d'une source malodorante solide ou liquide, proche dudit volume tridimensionnel, dans ledit volume tridimensionnel, et ladite source malodorante solide ou liquide dégageant la mauvaise odeur est choisie dans le groupe constitué par :

i. un herbicide malodorant ;
ii. une composition antivirale malodorante ;
iii. un fongicide malodorant ;
iv. un bactéricide malodorant;
v. un parasiticide malodorant;
vi. un insecticide malodorant ;
vii. une préparation dépilatoire malodorante ;
viii. une composition de blanchiment malodorante ;
ix. une préparation malodorante de nettoyage pour surface dure;
x. une composition malodorante de nettoyage de la peau ;
xi. une préparation malodorante anti-microbienne pour les ongles;
xii. une composition malodorante de fixation des cheveux ;
xiii. une composition malodorante de traitement des cheveux ;

xiv. une lotion trichologique malodorante ;
xv. une composition malodorante d'adoucissement de la peau ;
xvi. une composition détergente malodorante ;
xvii. une composition de savon malodorante ;
xviii. une composition malodorante d'écran solaire ;
xix. une composition malodorante de détachage des tissus ;
xx. une composition malodorante de traitement des tissus ;
xxi. une composition malodorante anti-froissement des tissus;
xxii. une composition aromatique malodorante pour fer à vapeur ;
xxiii. une composition de bougie malodorante ;
xxiv. une composition malodorante de régulation de la croissance des plantes ;
xxv. une composition malodorante de stimulation de la croissance des plantes ;
xxvi. une composition d'engrais malodorante ;
xxvii. une composition malodorante attirant les insectes;
xxviii. une composition malodorante répulsive des insectes;
xxix. une composition malodorante de nettoyage des tuyaux d'évacuation, et
xxx. une composition molluscicide malodorante.

**5.** Procédé selon la revendication 4, dans lequel la mauvaise odeur est provoquée par une quantité et une concentration provoquant une mauvaise odeur d'au moins un composé choisi dans le groupe constitué par des halohydrines aliphatiques, des amines aliphatiques, des N-oxydes aliphatiques, des dialkylamines, des amines cycloaliphatiques, des N-oxydes cycloaliphatiques, des amines cyclo-oléfiniques, des N-oxydes cyclo-oléfiniques, des amines cycloaromatiques, des N-oxydes cycloaromatiques, des hydroxyalkylamines, des composés imine, des composés amide, des acides aminés, des polypeptides, des protéines antimicrobiennes modifiées, des diuréides, des nitriles, des mercaptans aliphatiques, des mercaptans cycloaliphatiques, des acides mercaptoalcanoïques, des esters d'acides mercaptoalcanoïques, des monosulfures, disulfures, trisulfures, oxydes de soufre, sulfones et sultones aliphatiques, des monosulfures, disulfures, trisulfures, oxydes de soufre, sulfones et sultones cycloaliphatiques, des monosulfures, disulfures, trisulfures, oxydes de soufre, sulfones et sultones cyclo-oléfiniques, des monosulfures, disulfures, trisulfures, oxydes de soufre, sulfones et sultones cycloaromatiques, des isothiocyanates, des thiocyanates, des dithiocyanates, des isothiazolones, des isothiazolinones, des thiodiazinethiones, des halosulfamates, des arylsulfonamides, des acides carboxyliques aliphatiques inférieurs, des phénols, des phosphines, des phosphites et phosphonates aliphatiques, des phosphites et phosphonates cycloaliphatiques, des arsines, des alcools inférieurs, des cétones inférieures, l'opium, des acides d'opium, des arylpyrazoles, des oxazolines, des isocyanurates, des biguanides, des extraits de krameria, des hydantoïnes, des pyrrolidones, des acides pyrrolidone-carboxyliques, des esters d'acides pyrrolidone-carboxyliques, des nitrophénols, des acides aspartiques substitués en position N et des pyréthroïdes.

**6.** Procédé pour contrecarrer une mauvaise odeur, dans un savon ou détergent solide ou liquide contenant une fragrance, provoquée par une quantité et une concentration provoquant une mauvaise odeur d'au moins un composé choisi dans le groupe constitué par des halohydrines aliphatiques, des amines aliphatiques, des N-oxydes aliphatiques, des dialkylamines, des amines cycloaliphatiques, des N-oxydes cycloaliphatiques, des amines cyclo-oléfiniques, des N-oxydes cyclo-oléfiniques, des amines cycloaromatiques, des N-oxydes cycloaromatiques, des hydroxyalkylamines, des composés imine, des composés amide, des acides aminés, des polypeptides, des protéines antimicrobiennes modifiées, des diuréides, des nitriles, des mercaptans aliphatiques, des mercaptans cycloaliphatiques, des acides mercaptoalcanoïques, des esters d'acides mercaptoalcanoïques, des monosulfures, disulfures, trisulfures, oxydes de soufre, sulfones et sultones aliphatiques, monosulfures, disulfures, trisulfures, oxydes de soufre, sulfones et sultones cycloaliphatiques, des monosulfures, disulfures, trisulfures, oxydes de soufre, sulfones et sultones cyclo-oléfiniques, des monosulfures, disulfures, trisulfures, oxydes de soufre, sulfones et sultones cycloaromatiques, des isothiocyanates, des thiocyanates, des dithiocyanates, des isothiazolones, des isothiazolinones, des thiodiazinethiones, des halosulfamates, des arylsulfonamides, des acides carboxyliques aliphatiques inférieurs, des phénols, des phosphines, des phosphites et phosphonates aliphatiques, des phosphites et phosphonates cycloaliphatiques, des arsines, des alcools inférieurs, des cétones inférieures, l'opium, des acides d'opium, des arylpyrazoles, des oxazolines, des isocyanurates, des biguanides, des extraits de krameria, des hydantoïnes, des pyrrolidones, des acides pyrrolidone-carboxyliques, des esters d'acides pyrrolidonecarboxyliques, des nitrophénols, des acides aspartiques substitués en position N et des pyréthroïdes, comprenant l'étape consistant à introduire dans le savon ou détergent solide ou liquide une quantité et une concentration efficaces contrecarrant la mauvaise odeur de la composition selon la revendication 1, l'intensité d'odeur perçue de la fragrance contenue dans le savon ou le détergent solide ou liquide étant sensiblement maintenue.

**7.** Procédé pour contrecarrer une mauvaise odeur, dans un volume d'air tridimensionnel, tel que mesuré en utilisant les coordonnées "x", "y" et "z", contenant une fragrance, provoquée par une quantité et une concentration provoquant une mauvaise odeur, d'au moins un composé choisi dans le groupe constitué par des halohydrines aliphatiques, des amines aliphatiques, des N-oxydes aliphatiques, des dialkylamines, des amines cycloaliphatiques, des N-oxydes cycloaliphatiques, des amines cyclo-oléfiniques, des N-oxydes cyclo-oléfiniques, des amines cycloaromatiques, des N-oxydes cycloaromatiques, des hydroxyalkylamines, des composés imine, des composés amide, des acides aminés, des polypeptides, des protéines antimicrobiennes modifiées, des diuréides, des nitriles, des mercaptans aliphatiques, des mercaptans cycloaliphatiques, des acides mercaptoalcanoïques, des esters d'acides mercaptoalcanoïques, des monosulfures, disulfures, trisulfures, oxydes de soufre, sulfones et sultones aliphatiques, des monosulfures, disulfures, trisulfures, oxydes de soufre, sulfones et sultones cycloaliphatiques, des monosulfures, disulfures, trisulfures, oxydes de soufre, sulfones et sultones cyclo-oléfiniques, des monosulfures, disulfures, trisulfures, oxydes de soufre, sulfones et sultones cycloaromatiques, des isothiocyanates, des thiocyanates, des dithiocyanates, des isothiazolones, des isothiazolinones, des thiodiazinethiones, des halosulfamates, des arylsulfonamides, des acides carboxyliques aliphatiques inférieurs, des phénols, des phosphines, des phosphites et phosphonates aliphatiques, des phosphites et phosphonates cycloaliphatiques, des arsines, des alcools inférieurs, des cétones inférieures, l'opium, des acides d'opium, des arylpyrazoles, des oxazolines, des isocyanurates, des biguanides, des extraits de krameria, des hydantoïnes, des pyrrolidones, des acides pyrrolidone-carboxyliques, des esters d'acides pyrrolidonecarboxyliques, des nitrophénols, des acides aspartiques substitués en position N et des pyréthroïdes, comprenant l'étape consistant à introduire dans ledit volume d'air tridimensionnel, contenant une fragrance, tel que mesuré en utilisant les coordonnées "x", "y" et "z", une quantité et une concentration efficaces, contrecarrant la mauvaise odeur, de la composition selon la revendication 1, l'intensité d'odeur perçue de la fragrance contenue dans le volume d'air tridimensionnel, contenant une fragrance, tel que mesuré en utilisant les coordonnées "x", "y" et "z", étant sensiblement maintenue.

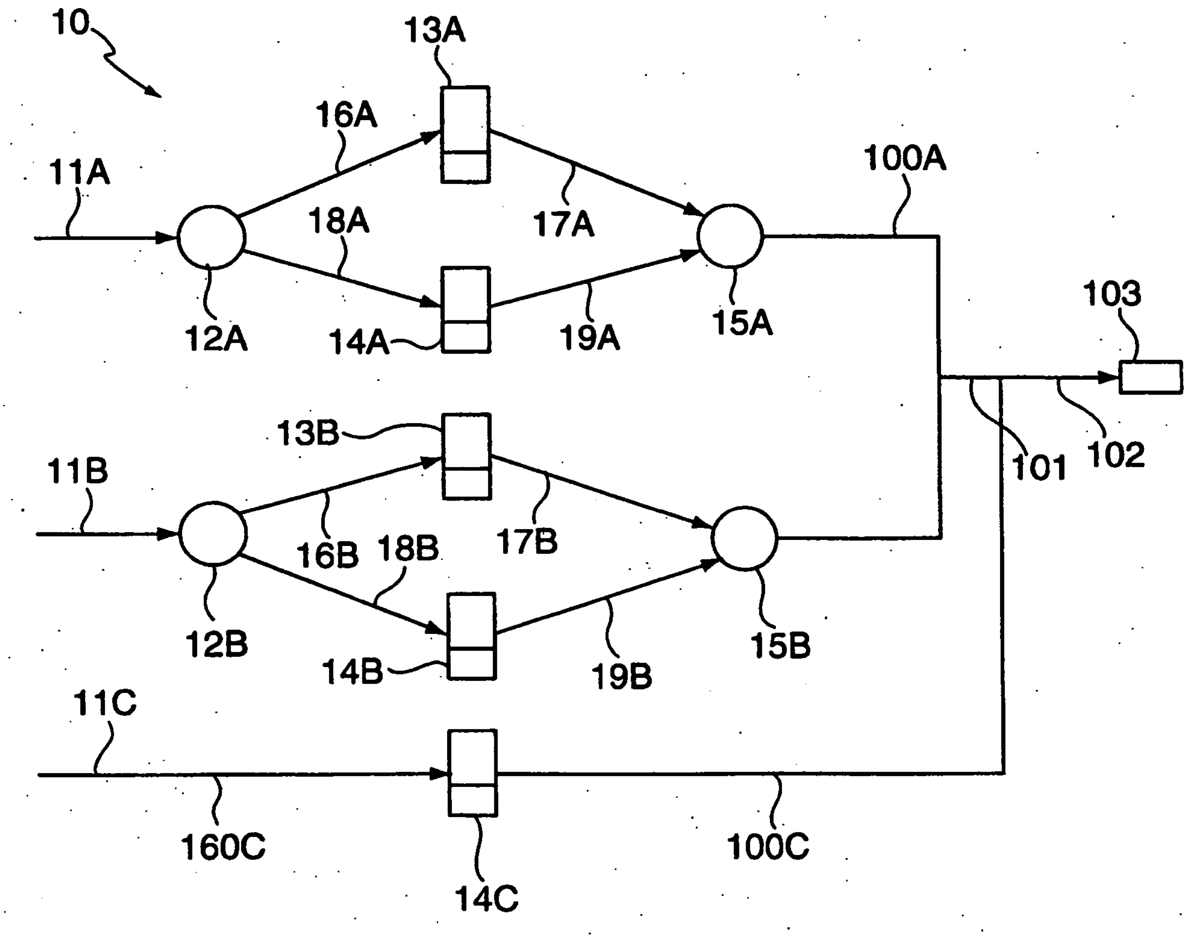
10
13A
16A
11A
100A
18A
17A
12A
14A
19A
15A
103
13B
11B
101
102
16B
18B
17B
12B
15B
14B
19B
11C
160C
100C
14C

FIG. 1

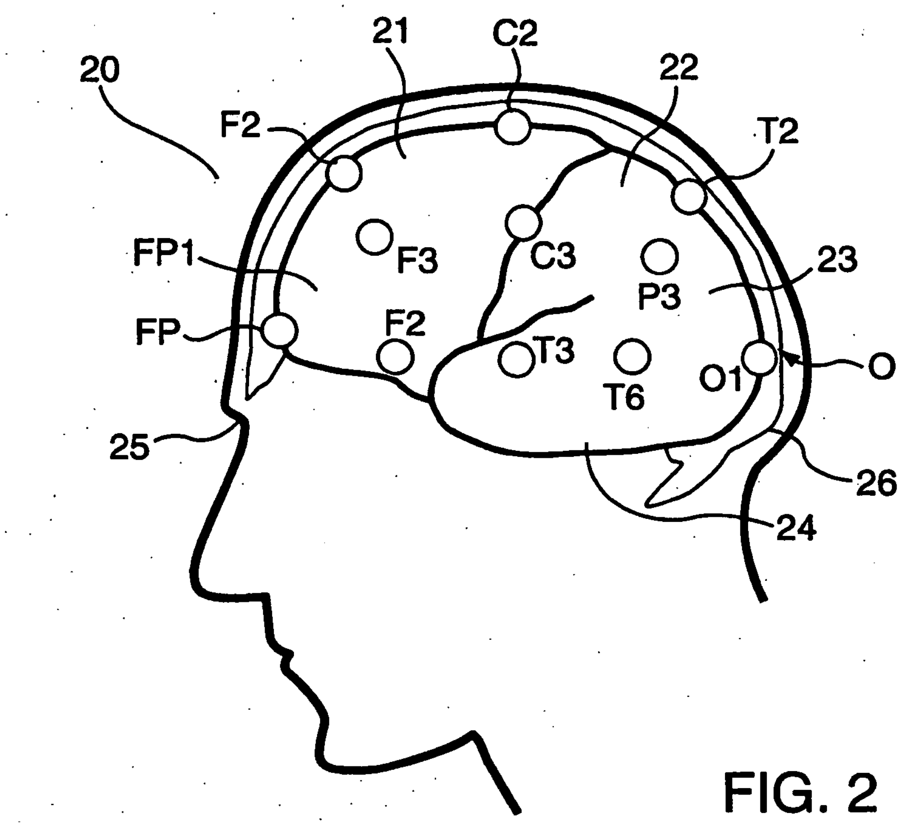
20
21
C2
22
F2
T2
F3
C3
FP1
23
P3
FP
F2
T3
O
T6
O1
25
26
24

FIG. 2

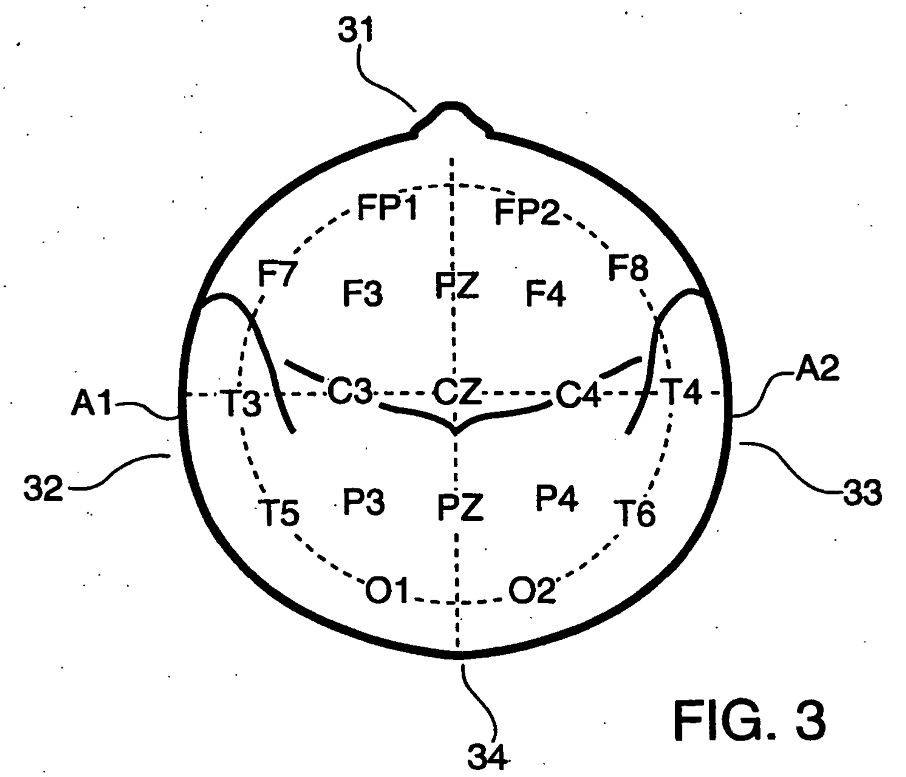
31
FP1
FP2
F7
F3
PZ
F4
F8
A1
T3
C3
CZ
C4
T4
A2
32
33
T5
P3
PZ
P4
T6
O1
O2
34

FIG. 3

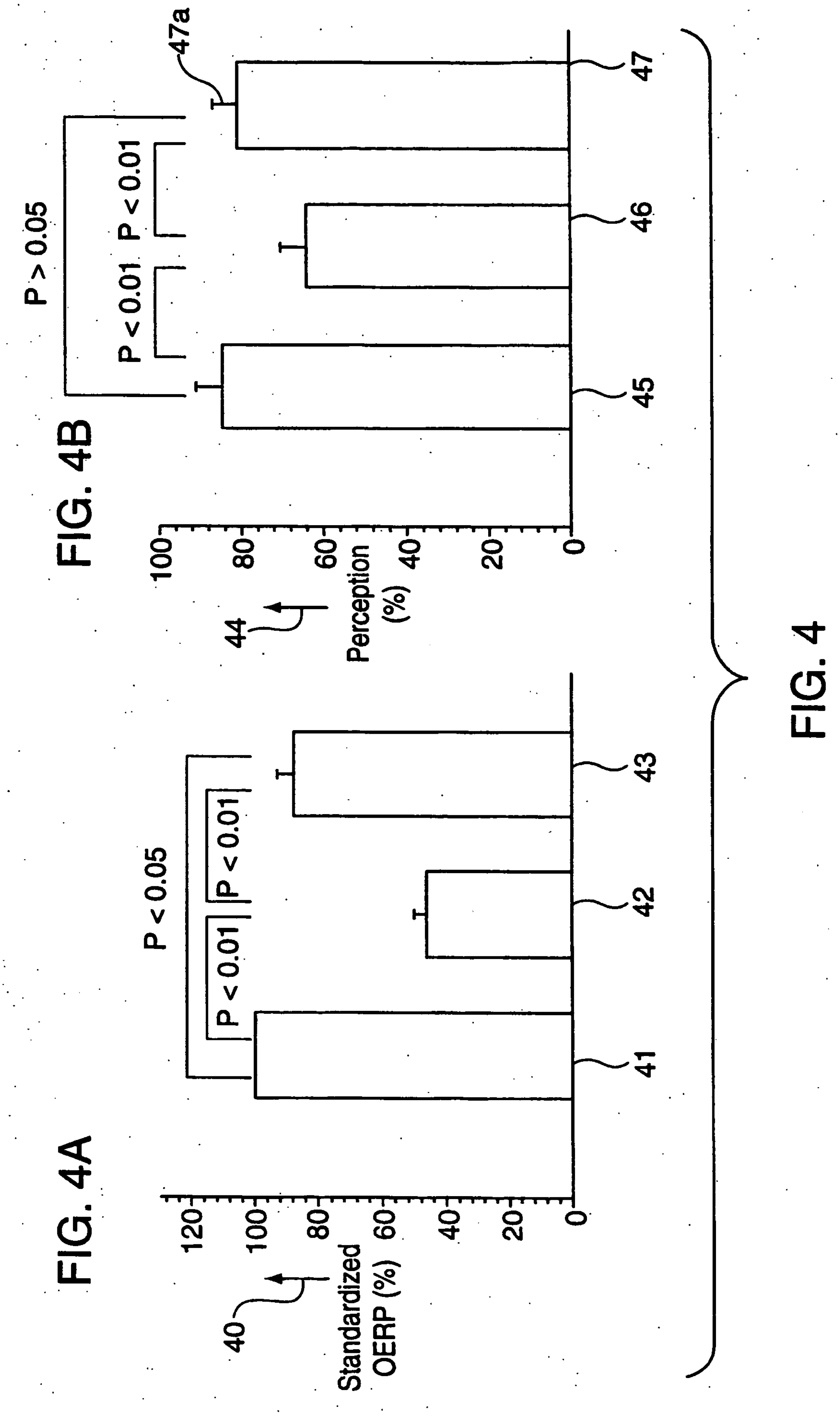
FIG. 4A
Standardized OERP (%)
40
120
100
80
60
40
20
0
P < 0.05
P < 0.01
P < 0.01
41
42
43
FIG. 4B
Perception (%)
44
100
80
60
40
20
0
P > 0.05
P < 0.01
P < 0.01
45
46
47
47a

FIG. 4

FIG. 5A

FIG. 5B

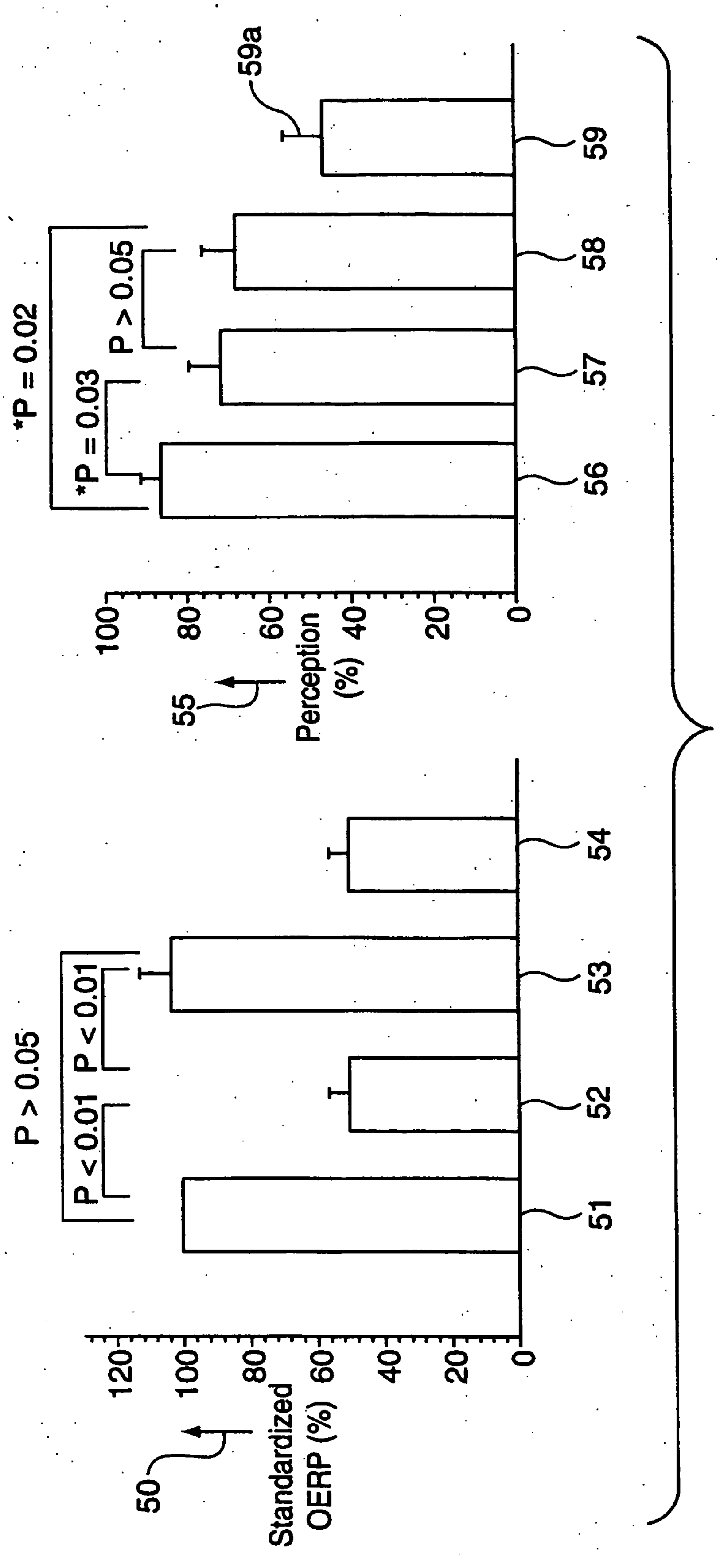


FIG. 5

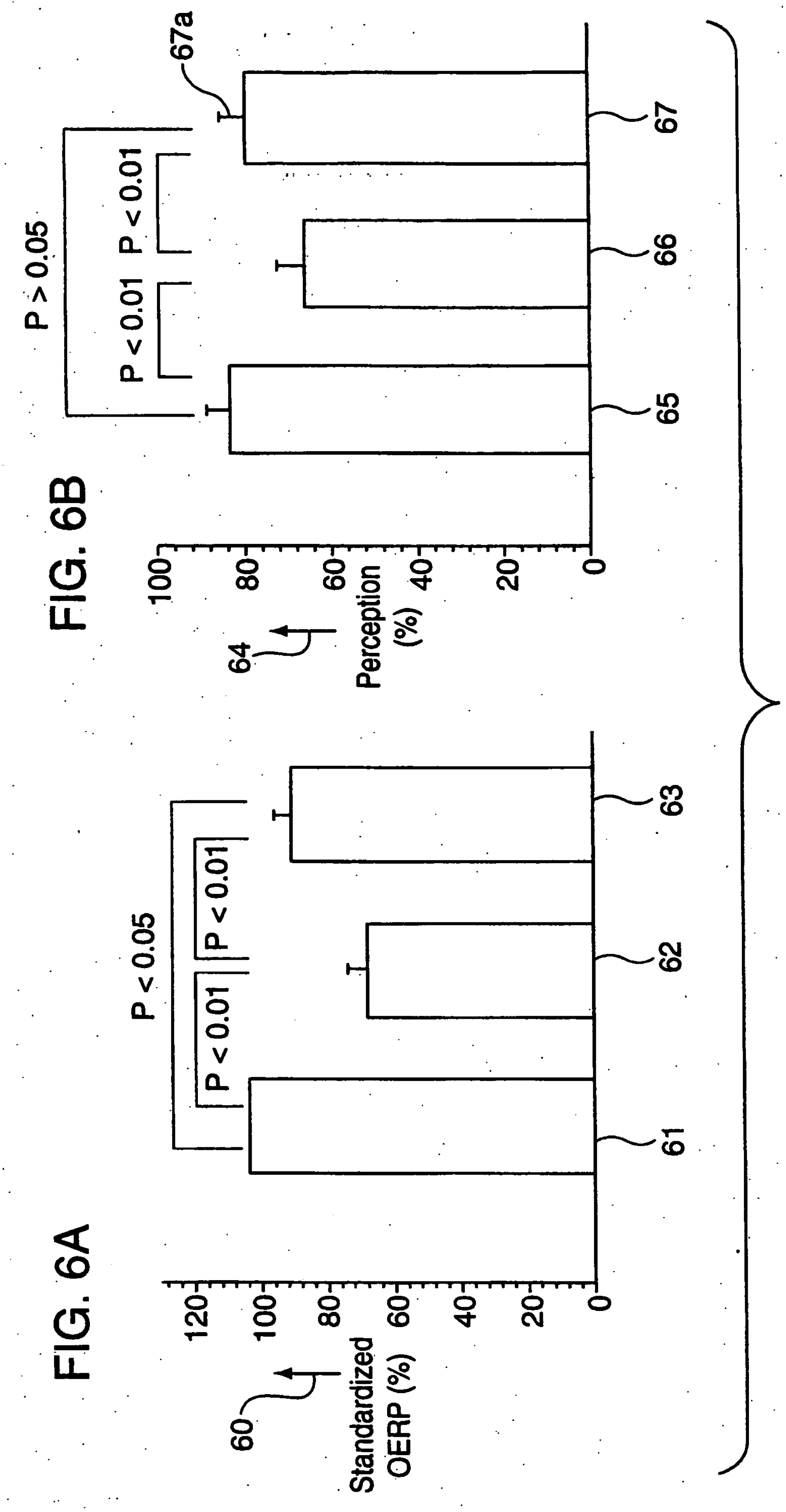
FIG. 6A
Standardized OERP (%)
60
120
100
80
60
40
20
0
P < 0.05
P < 0.01
P < 0.01
61
62
63
FIG. 6B
Perception (%)
64
100
80
60
40
20
0
P > 0.05
P < 0.01
P < 0.01
65
66
67
67a

FIG. 6

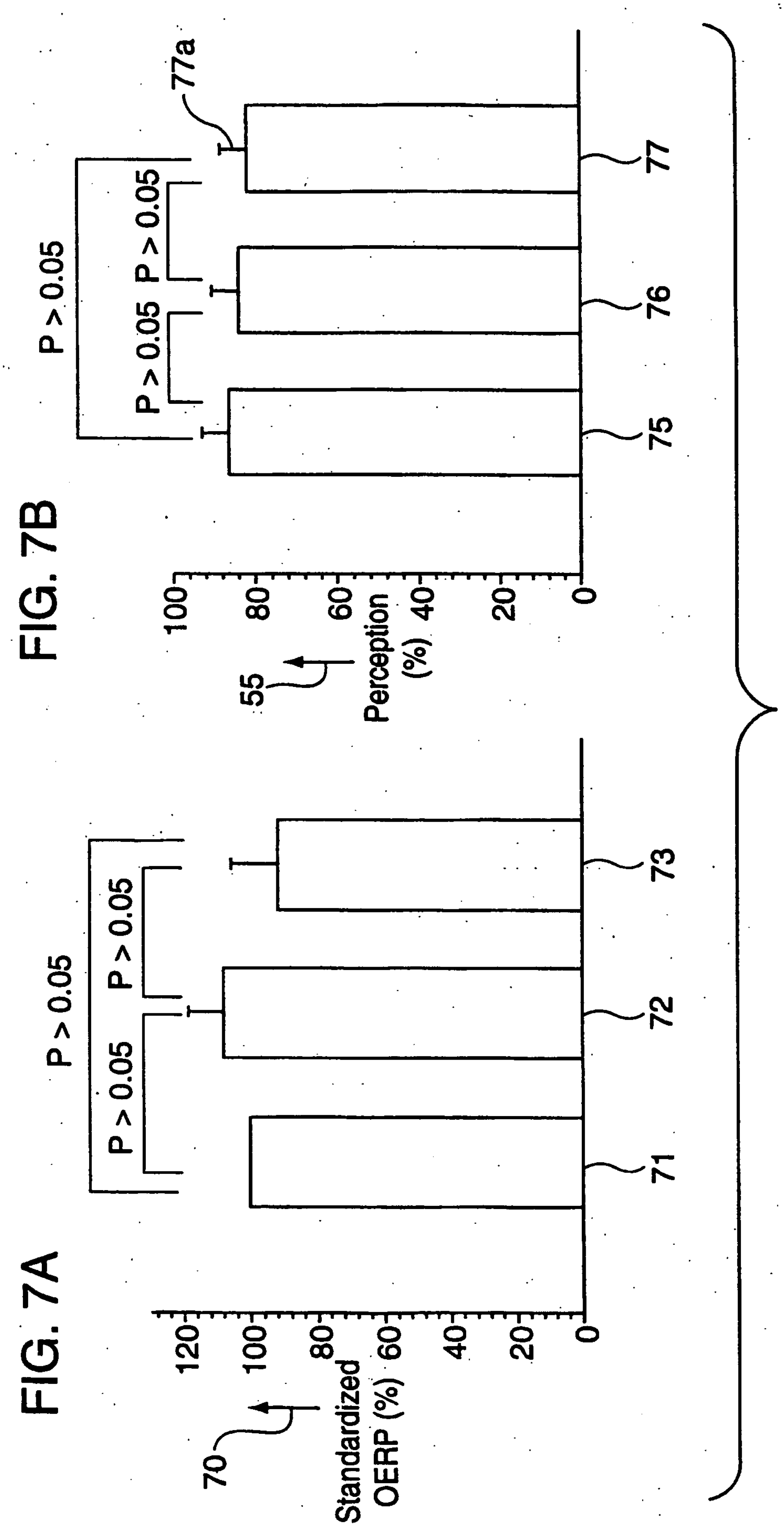
FIG. 7A
70
Standardized OERP (%)
0
20
40
60
80
100
120
P > 0.05
P > 0.05
P > 0.05
71
72
73
FIG. 7B
55
Perception (%)
0
20
40
60
80
100
P > 0.05
P > 0.05
P > 0.05
75
76
77
77a

FIG. 7

FIG. 8

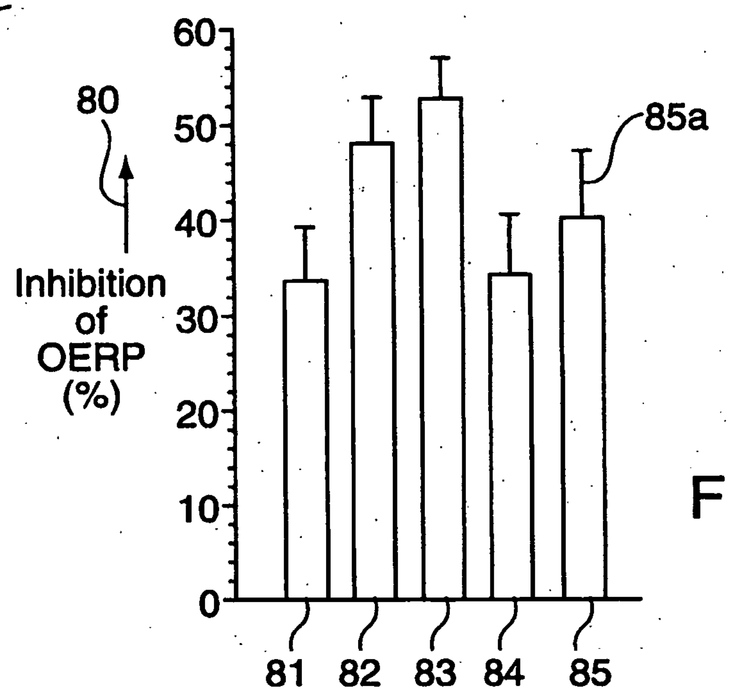


FIG. 8A

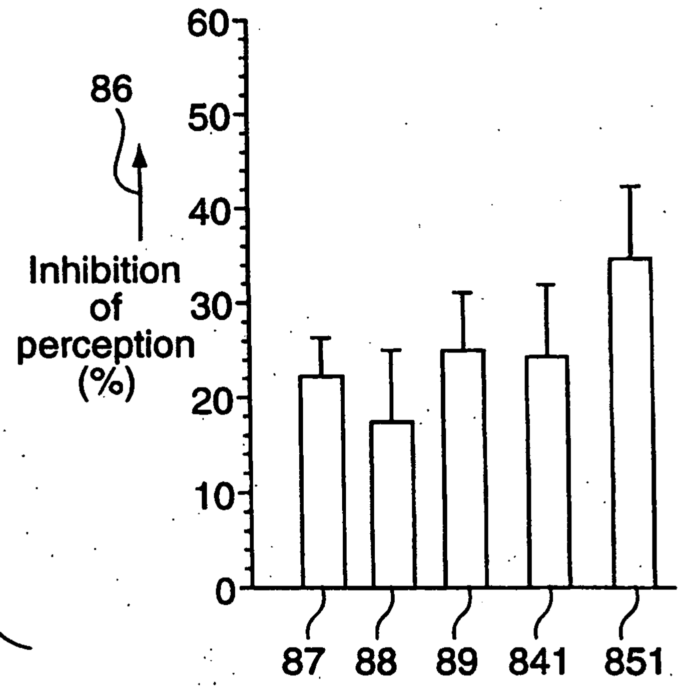


FIG. 8B

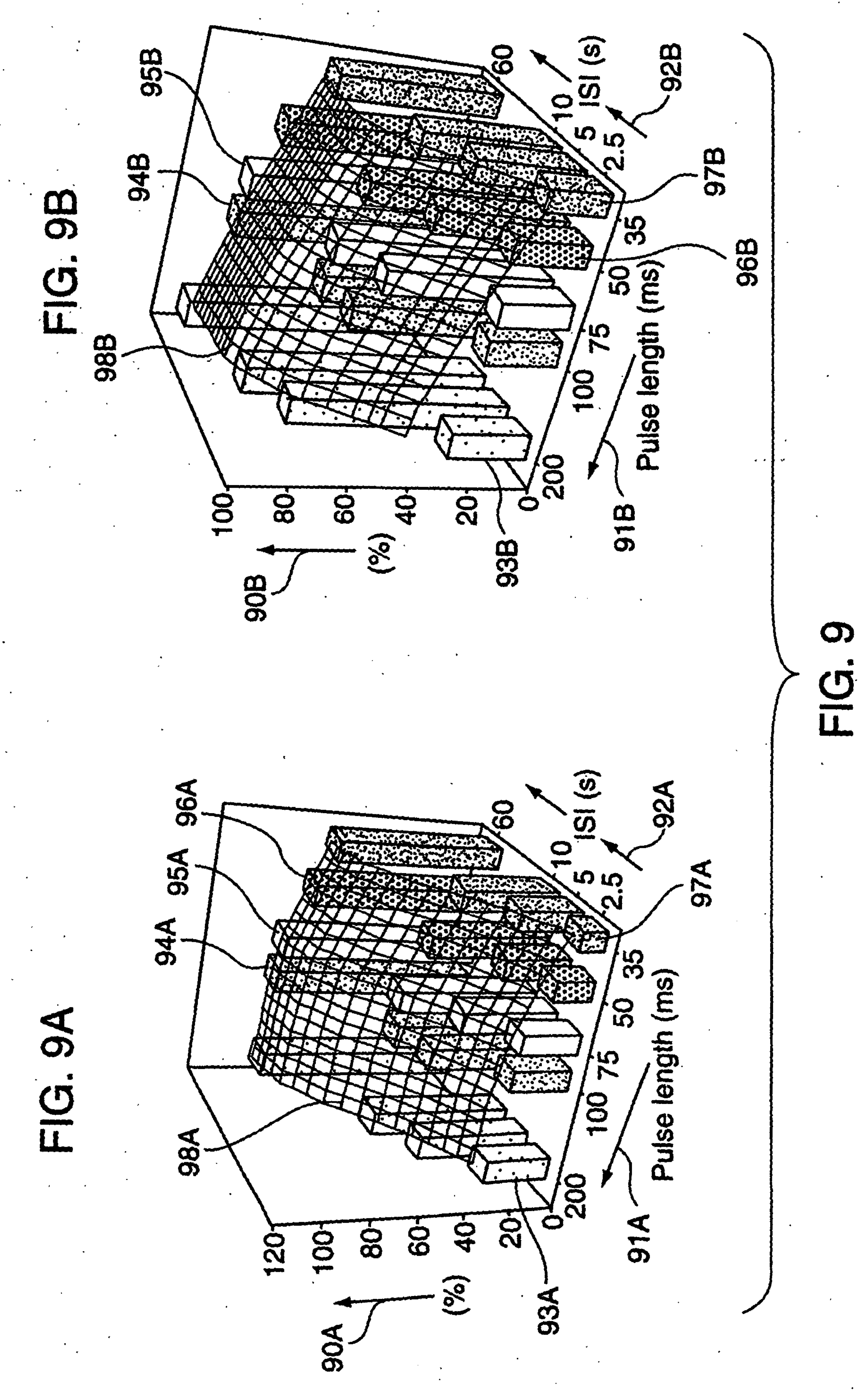
FIG. 9B
90B
(%)
100
80
60
40
20
0
200
100
75
50
35
2.5
5
10
60
ISI (s)
92B
Pulse length (ms)
91B
93B
94B
95B
96B
97B
98B
FIG. 9A
90A
(%)
120
100
80
60
40
20
0
200
100
75
50
35
2.5
5
10
60
ISI (s)
92A
Pulse length (ms)
91A
93A
94A
95A
96A
97A
98A

FIG. 9

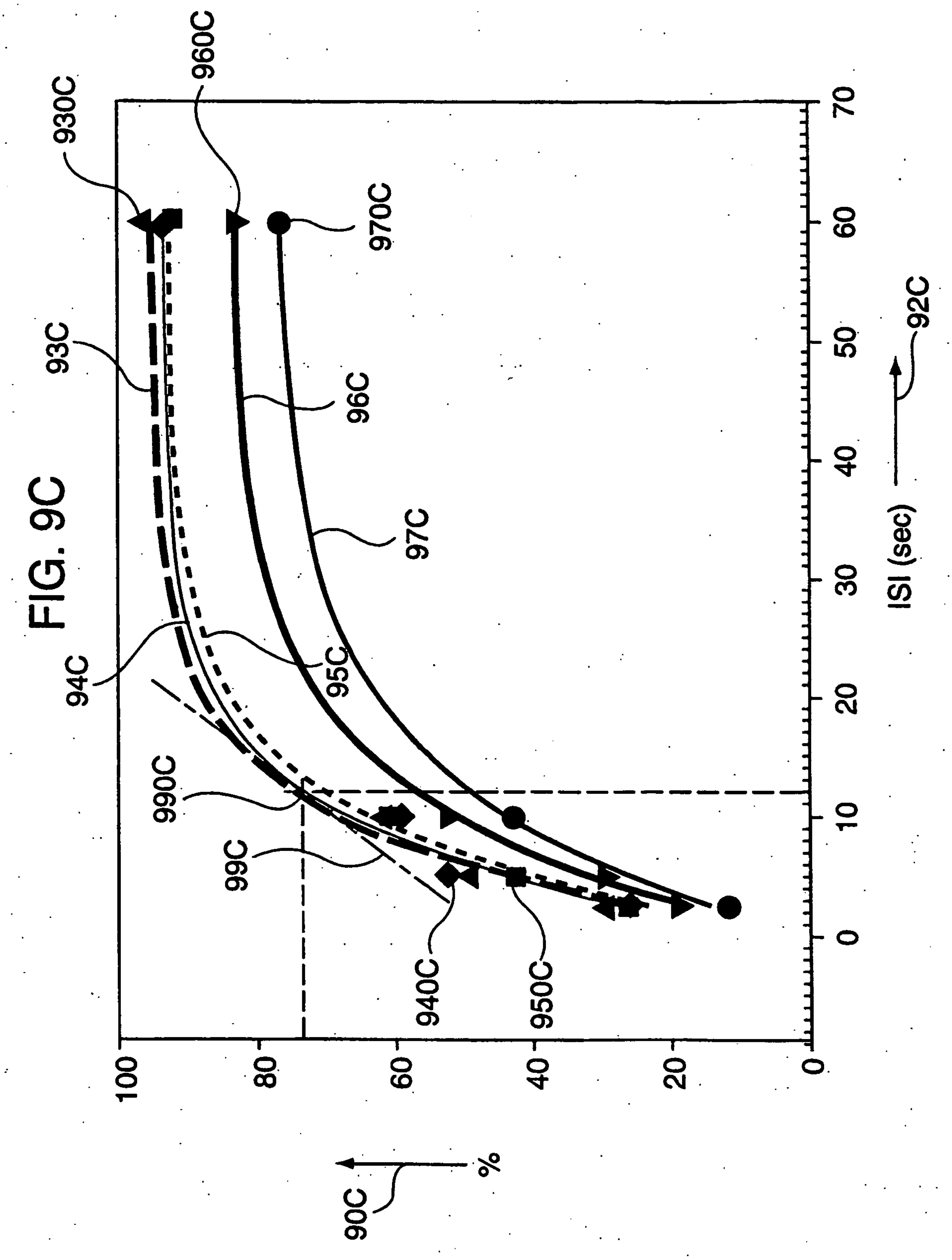
930C
960C
970C
93C
96C
97C
94C
95C
990C
99C
940C
950C
100
80
60
40
20
0
-10
0
10
20
30
40
50
60
70
ISI (sec)
92C
%
90C

FIG. 9C

FIG. 9D

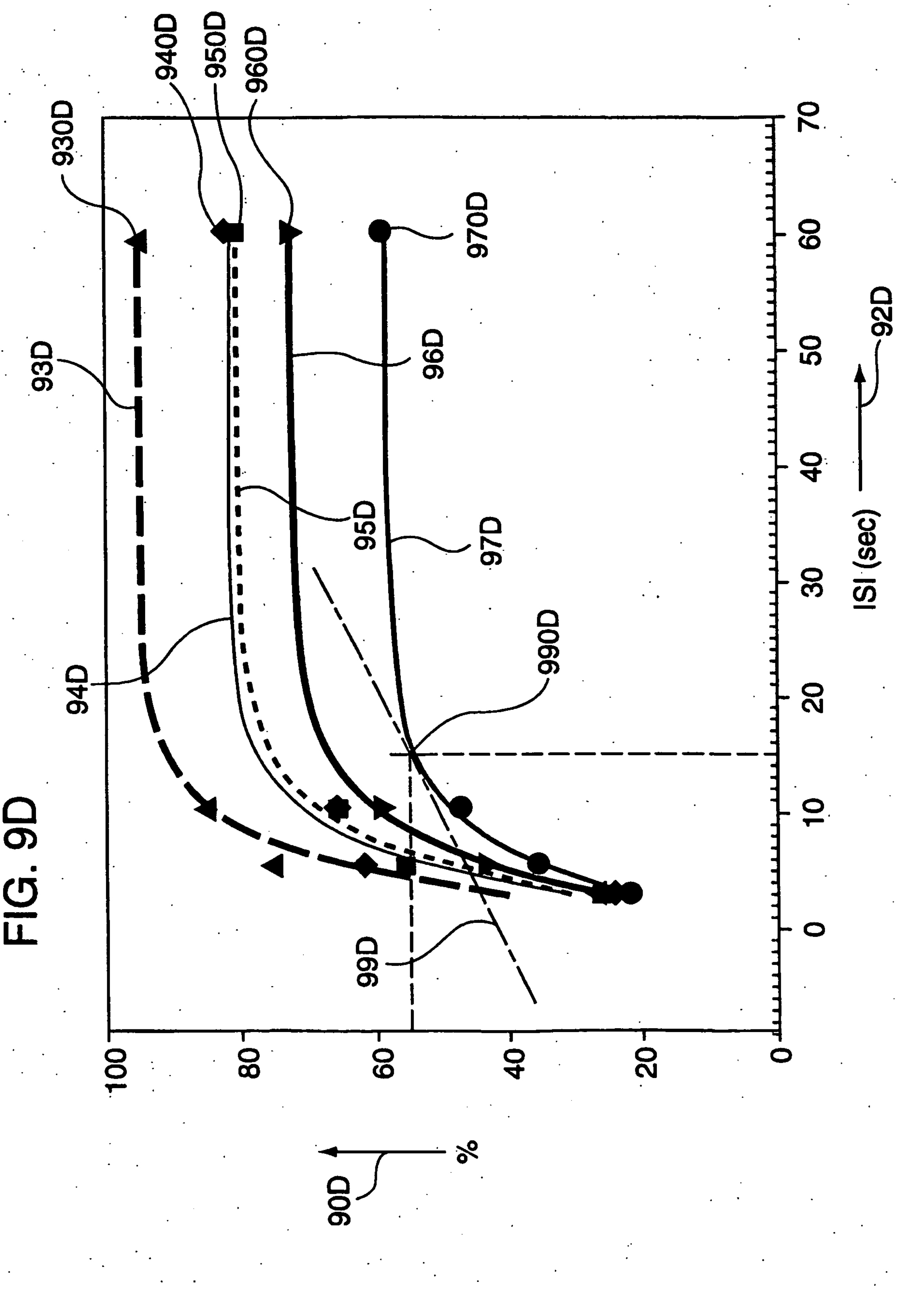
930D
940D
950D
960D
970D
93D
94D
95D
96D
97D
99D
990D
%
90D
100
80
60
40
20
0
-10
0
10
20
30
40
50
60
70
ISI (sec)
92D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4622221 A, Schleppnik
- US 6432891 B, O'Connor
- US 6325565 B
- US 10212349 B
- US 6491902 B
- US 6485736 B


### Non-patent literature cited in the description


- **JACOB et al.** Psychometric Evaluation of Responses to Pleasant and Malodour Stimulation in Human Subjects; Adaptation, Dose Response and Gender Differences. *Int. J. Psychophysiology,* 2003
- **WANG et al.** The correlation between physiological and psychological responses to odor stimulation in human subjects. *Clinical Neurophysiology,* 2002, vol. 113
- A Primer of Psychophysiology. **JAMES HASSET.** A Primer of Psychophysiology. 1978
- **WANG et al.** The correlation between physiological and psychological responses to odor stimulation in human subjects. *Clinical Neurophysiology,* 2002, vol. 113, 542-551